(19) Europäisches Patentamt
European Patent Office
Office européen des brevets

(11) EP 1 799 064 B1

(12) **FASCICULE DE BREVET EUROPEEN**

(45) Date de publication et mention de la délivrance du brevet:
**17.08.2016 Bulletin 2016/33**

(21) Numéro de dépôt: **05789860.3**

(22) Date de dépôt: **08.07.2005**

(51) Int Cl.:
***A45D 33/00*** *(2006.01)* ***A45D 34/00*** *(2006.01)*
***A45D 34/04*** *(2006.01)* ***A45D 29/00*** *(2006.01)*

(86) Numéro de dépôt international:
**PCT/FR2005/050563**

(87) Numéro de publication internationale:
**WO 2006/037905 (13.04.2006 Gazette 2006/15)**

(54) **PROCEDE DE MAQUILLAGE DES MATIERES KERATINIQUES ET KIT POUR LA MISE EN OEUVRE D'UN TEL PROCEDE**

MAKEUP-VERFAHREN FÜR KERATINMATERIAL UND AUSSTATTUNGSSATZ DAFÜR

METHOD FOR MAKING UP KERATINOUS MATERIALS AND KIT THEREFOR

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **05.10.2004 FR 0410501**
**20.10.2004 US 619928 P**

(43) Date de publication de la demande:
**27.06.2007 Bulletin 2007/26**

(73) Titulaire: **L'Oréal**
**75008 Paris (FR)**

(72) Inventeurs:
- **THEVENET, Ludovic**
**92340 BOURG LA REINE (FR)**
- **BLIN, Xavier**
**75015 Paris (FR)**

(74) Mandataire: **Nony**
**11 rue Saint-Georges**
**75009 Paris (FR)**

(56) Documents cités:
**EP-A- 1 264 562**
**WO-A-02/28356**
**WO-A2-2004/007096**
**FR-A- 2 851 463**
**US-A1- 2003 072 602**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 799 064 B1

## Description

La présente invention concerne le maquillage des matières kératiniques, notamment de la peau, des lèvres, des phanères, par exemple les cils ou les ongles.

La société demanderesse a développé en interne des compositions de maquillage, en particulier des lèvres, qui comportent des particules magnétiques, lesquelles sont susceptibles en présence d'un champ magnétique de s'orienter et/ou de se déplacer, et de conduire à de nouveaux effets optiques.

Un problème qui se pose avec ces compositions est la persistante dans le temps de l'effet obtenu.

EP 1 264 562 divulgue des compositions comportant des particules magnétiques, appliquées au moyen d'applicateurs magnétiques.

L'invention vise à proposer une composition à appliquer sur les matières kératiniques, notamment la peau ou les lèvres, permettant l'obtention d'un résultat durable.

### PROCEDE DE MAQUILLAGE

L'invention ainsi pour objet, selon l'un de ses aspects, un procédé de maquillage des matières kératiniques, notamment de la peau et des lèvres, comprenant les étapes suivantes :

(a) appliquer sur les matières kératiniques au moyen d'un applicateur cosmétique non magnétique au moins une composition de maquillage comprenant

(i) au moins un solvant volatil, notamment une huile volatile,
(ii) des particules ayant une susceptible magnétique non nulle, et

(b) soumettre le dépôt à un champ magnétique de manière à modifier l'orientation et/ou à déplacer au moins certaines desdites particules à susceptibilité magnétique non nulle, le champ magnétique étant appliqué de manière à former au moins un motif sur la composition.

La présence d'au moins un solvant volatil, notamment une huile volatile, est avantageuse en ce qu'elle permet d'une part une certaine mobilité des particules magnétiques, immédiatement après l'application, sous l'effet d'un champ magnétique, et d'autre part l'immobilisation de ces particules au terme d'une certaine durée de séchage dans l'orientation qui leur a été conférée.

Avantageusement, la composition comprend au moins un polymère filmogène, ce qui peut améliorer encore l'immobilisation des particules après séchage.

Le champ magnétique peut être appliqué de manière à former au moins un motif sur la composition, celui-ci étant par exemple lié à la géométrie des lignes de champ.

Le cas échéant, une couche d'une deuxième composition cosmétique peut être appliquée sur celle contenant les corps magnétiques, par exemple en vue d'obtenir un effet de profondeur, de brillance, de lissage ou autre. Cette deuxième composition peut être transparente, colorée ou non. La deuxième composition peut encore être appliquée sur le support avant la première composition, par exemple pour créer un fond coloré ou améliorer la tenue de la première composition et/ou le confort.

Le champ magnétique peut être appliqué jusqu'à obtenir un aspect figé de la composition contenant les corps magnétiques, c'est-à-dire que l'aspect de celle-ci cesse d'évoluer même si le champ magnétique perdure. En variante, le champ magnétique peut être appliqué pendant une durée inférieure à celle provoquant l'orientation et/ou le déplacement définitif de la totalité des corps magnétiques de la région exposée. Lorsque la clarté et/ou la couleur de la composition changent progressivement sous l'effet du champ magnétique, l'utilisateur peut alors arrêter de soumettre les corps magnétiques au champ lorsque la composition présente l'aspect souhaité.

Le champ magnétique peut être exercé successivement sur différentes régions du support revêtues de la composition.

Le champ magnétique peut être exercé sur des régions disjointes du support, afin par exemple de créer des motifs séparés.

Une région du support revêtue de la composition peut être non exposée au champ magnétique, de manière à ne pas modifier dans cette région l'aspect de la composition après son dépôt.

Deux régions du support peuvent être exposées de manière inégale au champ magnétique.

La composition peut être appliquée de diverses manières au moyen d'un applicateur cosmétique non magnétique, choisi par exemple parmi les pinceaux, les embouts floqués, les mousses, les tissés, les non-tissés, les brosses ou peignes.

L'invention a encore pour objet un kit pour la mise en oeuvre du procédé précité.

Un tel kit peut comporter :

- une composition de maquillage comprenant :

  (i) au moins un solvant volatil, notamment une huile volatile,
  (ii) des particules ayant une susceptibilité magnétique non nulle,

- un applicateur cosmétique non magnétique,
- un dispositif magnétique permettant de générer un champ magnétique,
- le dispositif magnétique étant apte à créer un champ magnétique susceptible, lorsque les matières kératiniques recouvertes d'un dépôt de ladite composition sont introduites dans ledit champ magnétique, de modifier l'orientation et/ou la position des corps magnétiques à l'intérieur du dépôt, le champ magnétique étant appliqué de manière à former au moins un motif sur la composition.

Avantageusement, la composition comporte en outre au moins un polymère filmogène.
Le dispositif magnétique peut comporter au moins un aimant permanent ou un électroaimant, alimenté par exemple par au moins une pile ou un accumulateur. Dans ce dernier cas, le dispositif magnétique peut comporter un interrupteur permettant d'alimenter sélectivement l'électroaimant en électricité.
Le dispositif magnétique du kit peut être agencé pour créer un champ magnétique dont l'orientation varie dans le temps. Lorsque le dispositif magnétique comporte un aimant, le dispositif peut par exemple comporter un moteur permettant d'entraîner en rotation l'aimant. En variante, le dispositif magnétique peut comporter plusieurs solénoïdes disposés de manière à générer, lorsqu'alimentés séquentiellement en électricité, un champ magnétique tournant.
Un champ magnétique rotatif peut permettre par exemple d'obtenir un motif présentant une symétrie de révolution, par exemple un motif donnant l'impression d'une sphère en relief.
Le ou les électroaimants peuvent être alimentés en permanence ou par intermittence, au choix de l'utilisateur. En particulier, le dispositif magnétique du kit peut être agencé de telle sorte que le ou les électroaimants puissent ne pas être alimentés tant que le dispositif magnétique n'est pas positionné correctement près du support revêtu de la composition.
Le champ magnétique est par exemple d'au moins 50 mT, voire d'au moins 0,2 T ou 1T.
De manière à rendre plus facile l'application du champ magnétique, le dispositif magnétique du kit peut comporter un organe permettant de le positionner relativement au support sur lequel la composition a été déposée. Cela peut permettre par exemple d'éviter que le dispositif magnétique ne vienne accidentellement en contact avec la composition et/ou de centrer le motif réalisé sur la région concernée.
Le dispositif magnétique du kit peut être solidaire d'un applicateur servant à l'application de la composition cosmétique. Cela peut permettre de réduire le nombre d'objets manipulés par l'utilisateur et faciliter le maquillage.
Le dispositif magnétique du kit peut comporter un aimant monté à une première extrémité d'une tige dont la deuxième extrémité est reliée à un organe de préhension d'un applicateur servant à l'application de la composition cosmétique.
Le champ magnétique peut encore être exercé au moyen d'une structure magnétique, notamment souple, comportant une alternance de pôles N et S. Une telle structure peut permettre par exemple de réaliser des motifs répétitifs sur la composition, par exemple des rayures.
Le kit peut comporter un boîtier logeant la composition cosmétique et le dispositif magnétique. Dans ce cas, le boîtier peut comporter par exemple une pluralité d'aimants de formes différentes pour réaliser des motifs différents.
La composition cosmétique comporte :

- au moins un solvant volatil, notamment une huile volatile,
- des corps magnétiques comportant du fer métal, éventuellement enrobé, notamment du fer doux.

Avantageusement, la composition comporte au moins un polymère filmogène.
La présence de fer métal confère aux corps magnétiques une sensibilité élevée au champ magnétique.
L'invention pourra être mieux comprise à la lecture de la description détaillée qui va suivre, d'exemples de mise en oeuvre non limitatifs de celle-ci, et à l'examen du dessin annexé, sur lequel :

- la figure 1 représente de manière schématique un exemple de kit pour la mise en oeuvre du procédé,
- la figure 2 illustre l'application de la composition sur les lèvres, et
- la figure 3 illustre l'exposition de la composition à un champ magnétique.

Le kit 1 représenté à la figure 1 comporte un récipient 2 contenant une composition fluide C à appliquer sur

les lèvres, et un applicateur 3 comportant un organe d'application 4 monté à l'extrémité d'une tige 5 dont l'autre extrémité est reliée à un organe de préhension 6 qui constitue également un capuchon de fermeture du récipient 2.

Le récipient 2 est muni d'un organe d'essorage 7 de la tige 5 et de l'organe d'application 4, de façon conventionnelle.

Le kit 1 comporte en outre un dispositif magnétique 10 qui est par exemple constitué par un aimant permanent, mais qui pourrait dans une variante non illustrée comporter au moins un électroaimant ou un aimant solidaire d'un système mécanique ou électromécanique l'entraînant en mouvement de manière prédéfinie, de façon à créer un motif ayant la forme voulue sur le dépôt de la composition à effectuer.

Le kit 1 s'utilise en appliquant dans un premier temps la composition C au moyen de l'applicateur 3, comme illustré à la figure 2, par exemple sous la forme d'une ou plusieurs couches fines, puis, comme illustré à la figure 3, en exposant dans un deuxième temps la composition ainsi déposée, avant qu'elle ne sèche, à un champ magnétique de manière à permettre la formation du motif souhaité.

Conformément à un aspect de l'invention, la composition contient au moins un solvant volatil.

## Solvants volatils

Au sens de la présente invention, on entend par « solvant volatil», un solvant, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

D'une manière générale, la quantité de solvant(s) dépendra de la nature du support sur lequel la composition est destinée à être appliquée.

Le solvant peut être choisi parmi l'eau, les solvants organiques et les huiles.

L'huile peut être une huile siliconée ou une huile hydrocarbonée, ou comporter un mélange de telles huiles.

Au sens de la présente invention, on entend par « huile siliconée », une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS® ou de PERMETHYLS®.

Comme huiles volatiles, on peut aussi utiliser les silicones volatiles, comme par exemple les huiles de silicones linéaires ou cycliques volatiles, notamment celles ayant une viscosité $\leq$ 8 centistokes (8 x $10^{-6}$ $m^2/s$), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

La composition selon l'invention peut comprendre entre 0,01 % et 95% en poids d'huile volatile, par rapport au poids total de la composition, mieux entre 1 % et 75 % en poids.

La composition peut comporter au moins un solvant organique choisi dans la liste suivante :

- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle.

La composition peut aussi comprendre de l'eau ou un mélange d'eau et de solvants organiques hydrophiles

couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols, des éthers en $C_2$ et des aldéhydes en $C_2$-$C_4$ hydrophiles.

L'eau ou le mélange d'eau et de solvants organiques hydrophiles peut être présent dans la composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

Conformément à un aspect de l'invention, la composition appliquée contient au moins un polymère filmogène.

## Polymère filmogène

Par polymère "filmogène", on peut entendre un polymère apte à former à lui seul ou en présence d'un agent auxiliaire de filmification, un film continu sur un support, notamment sur les matières kératiniques, et de préférence un film cohésif et mieux encore, un film dont la cohésion et les propriétés mécaniques sont telles que ledit film peut être isolé dudit support.

Parmi les polymères filmogènes utilisables dans la composition de la présente invention, on peut citer les polymères synthétiques, de type radicalaire ou de type polycondensat, les polymères d'origine naturelle et leurs mélanges.

Comme polymère filmogène, on peut citer en particulier les polymères acryliques, les polyuréthanes, les polyesters, les polyamides, les polyurées, les polymères cellulosiques comme la nitrocellulose.

Ces polymères filmogènes peuvent être distingués en quatre classes, en fonction de leur solubilité à l'égard d'une phase aqueuse ou d'une phase grasse liquide.

Dans un exemple de mise en oeuvre de réalisation, le polymère filmogène est au moins un polymère choisi parmi le groupe comprenant :

- les polymères filmogènes solubles dans la phase grasse liquide de la composition, en particulier les polymères liposolubles,
- les polymères filmogènes dispersibles dans la phase grasse liquide de la composition, en particulier les polymères sous la forme de dispersions non aqueuses de particules de polymères, en particulier des dispersions dans les huiles siliconées ou hydrocarbonées;
- les dispersions aqueuses de particules de polymères filmogènes, souvent appelées « latex » ; dans ce cas, la composition doit comprendre, outre la phase grasse liquide, une phase aqueuse,
- les polymères filmogènes hydrosolubles ; dans ce cas également, la composition doit comprendre, outre la phase grasse liquide, une phase aqueuse.

Selon un autre mode de réalisation de l'invention, le polymère filmogène est siliconé et peut être choisi parmi les polymères ayant un squelette organique non siliconé greffé par des monomères contenant un polysiloxane.

Selon un autre mode de réalisation de l'invention, le polymère filmogène est siliconé et est choisi parmi les polymères siliconés greffés par des monomères organiques non siliconés. Ces polymères peuvent être liposolubles, lipodispersables, hydrosolubles ou dispersables en milieu aqueux, le cas échéant.

Pour des raisons évidentes, les quantités en agent filmogène au sein des compositions selon l'invention, sont susceptibles de varier significativement notamment au regard de la nature de l'agent filmogène considéré et d'autre part des qualités recherchées au niveau de la composition l'incorporant.

Ainsi, dans les compositions cosmétiques conformes à l'invention, la teneur en polymère(s) filmogène(s) peut varier de 0,01 à 65 % en poids, notamment de 0,1 à 60 % en poids, et en particulier de 1 à 45 % en poids, par rapport au poids total de la composition.

La composition peut comprendre, à titre de polymère, une dispersion de particules d'un polymère éthylénique greffé dans une phase grasse liquide.

Par polymère "éthylénique", on entend un polymère obtenu par polymérisation de monomères comprenant une insaturation éthylénique.

La dispersion de polymère éthylénique greffé est notamment exempte de polymère stabilisant distinct dudit polymère greffé, tels que ceux décrits dans EP749747 et décrits plus loin, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules.

Par polymère "greffé", on entend un polymère ayant un squelette comprenant au moins une chaîne latérale pendante ou située en bout de chaîne, et de préférence pendante.

Avantageusement, le polymère éthylénique greffé comprend un squelette éthylénique insoluble dans la phase grasse liquide, et des chaînes latérales liées de manière covalente audit squelette et solubles dans la phase grasse liquide.

Le polymère éthylénique greffé est notamment un polymère non réticulé. En particulier, le polymère est obtenu par polymérisation de monomères comprenant un seul groupement polymérisable.

Le polymère éthylénique greffé est par exemple un polymère acrylique greffé.

Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation du polymère éthylénique greffé.

Le milieu organique liquide de dispersion, correspondant au milieu dans lequel est fourni le polymère greffé, peut être identique au milieu de polymérisation.

Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.

La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.

Le milieu liquide organique de dispersion peut être présent dans la phase grasse liquide de la composition selon l'invention du fait de l'introduction dans la composition de la dispersion de polymère greffé obtenue.

La phase grasse liquide comprend, de préférence majoritairement, un ou plusieurs composés organiques liquides (ou huiles) tels que définis ci-après.

En particulier, la phase grasse liquide peut être une phase organique liquide non aqueuse et non miscible à l'eau à la température ambiante (25 °C).

On entend par "composé organique liquide" un composé non aqueux qui est à l'état liquide à la température ambiante (25 °C) et qui s'écoule donc de son propre poids.

Parmi les composés organiques liquides ou huiles pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Le paramètre de solubilité global $\delta$ selon l'espace de solubilité de Hansen est défini dans l'article « Solubility parameter values » de Eric A.Grrulke de l'ouvrage « Polymer Handbook », 36éme édition, Chapitre VII, p.519-559 par la relation :

$$\delta = (\delta_D{}^2 + \delta_P{}^2 + \delta_H{}^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents, et
- $d_H$ caractérise les forces d'interactions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides organiques, notamment non siliconés ou siliconés, ayant un paramètre de solubilité global selon l'espace de solubilité de Hansen inférieur ou égal à 18 $(Mpa)^{1/2}$ , on peut citer des corps gras liquides, notamment des huiles, qui peuvent être choisis parmi les huiles naturelles ou synthétiques, carbonées, hydrocarbonées,

fluorées, siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones.

On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, on peut citer les huiles de silicone, éventuellement ramifiées, volatiles et/ou non volatiles.

On peut citer, en particulier, comme composés organiques liquides non siliconés ayant un paramètre de solubilité global selon l'espace de solubilité de Hansen inférieur ou égal à 18 $(Mpa)^{1/2}$ :

- les esters linéaires, ramifiés ou cycliques, ayant au moins 6 atomes de carbone, notamment de 6 à 30 atomes de carbone ;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, le décanol, l'octyldodécanol et l'alcool linoléique.

Lorsque la phase grasse liquide de la composition est une phase grasse liquide non siliconée, les macromonères présents dans le polymère greffé sont avantageusement des macromonomères carbonés tels que décrits ci-après.

En particulier, lorsque la phase grasse liquide de la composition est une phase grasse liquide non siliconée, le polymère greffé présent dans la composition est avantageusement un polymère greffé non siliconé.

Par polymère greffé non siliconé, on entend un polymère greffé contenant majoritairement un macromonomère carboné et contenant éventuellement au plus 7 % en poids, de préférence au plus 5 % en poids, voire est exempt, de macromonomère siliconé.

Lorsque la phase grasse liquide de la composition cosmétique selon l'invention est une phase grasse liquide siliconée, les macromonomères présents dans le polymère greffé sont avantageusement des macromonomères siliconés tels que décrits ci-après.

En particulier, lorsque la phase grasse liquide est une phase grasse liquide siliconée, le polymère greffé présent dans la composition est avantageusement un polymère greffé siliconé.

Par polymère greffé siliconé, on entend un polymère greffé contenant majoritairement un macromonomère siliconé et contenant éventuellement au plus 7 % en poids, de préférence au plus 5 % en poids, voire est exempt, de macromonomère carboné.

### *a) __Monomères__*

Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Le polymère éthylénique greffé formant les particules en dispersion comprend donc un squelette insoluble dans ledit milieu de dispersion et une partie soluble dans ledit milieu de dispersion.

Le polymère éthylénique greffé peut être un polymère statistique.

Selon l'invention, on entend par "polymère éthylénique greffé " un polymère susceptible d'être obtenu par polymérisation radicalaire :

- d'un ou plusieurs monomère(s) éthylénique(s),

- avec un ou plusieurs macromonomère(s), dans un milieu organique de polymérisation.

Selon l'invention, on entend par "polymère acrylique greffé " un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

En particulier, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

En particulier, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersion considéré alors que les chaînes latérales (ou greffons) sont solubles dans ledit milieu de dispersion.

Par "monomère acryliques", on entend dans la présente demande des monomères choisis parmi l'acide (méth)acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule (VIII) :

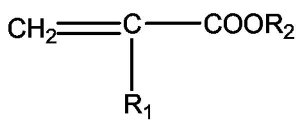

(VIII)

dans laquelle :

- $R_1$ désigne un atome d'hydrogène ou un groupe méthyle ;
- $R_2$ représente un groupe choisi parmi :
    - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; et/ou pouvant être substitué

par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;

- un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE , trifluoroéthyle, 2-hydroxyéthyle, 2-hydroxypropyle, diméthylaminoéthyle, diéthylaminoéthyle, diméthylaminopropyle.

- (ii) les (méth)acrylamides de formule (IX) :

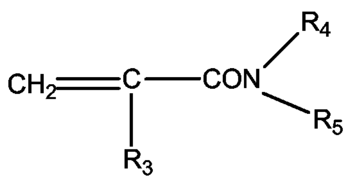

(IX)

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$ ; ou
- $R_4$ représente un atome d'hydrogène et $R_5$ représente un groupe 1,1-diméthyl-3-oxobutyle.

A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle :

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels ; et leurs mélanges.

En particulier, les monomères acryliques sont choisis parmi l'acrylate de méthyle, l'acrylate de méthoxyéthyle, le méthacrylate de méthyle, le méthacrylate de 2-hydroxyéthyle, l'acide acrylique, le méthacrylate de diméthylaminoéthyle, et leurs mélanges.

Parmi les monomères additionnels vinyliques non acryliques, on peut citer :

- les esters vinyliques de formule suivante :

$$R_6\text{-COO-CH=CH}_2$$

dans laquelle :

- $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;

- les monomères vinyliques non acryliques comprenant au moins une fonction amine tertiaire, tels que la 2-vinylpyridine, la 4-vinylpyridine ;
- et leurs mélanges.

Avantageusement, les monomères acryliques présents dans le polymère greffés comprennent au moins l'acide (méth)acrylique et au moins un monomère choisi parmi les (méth)acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.
Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.
On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.
Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.
Il est entendu que ces monomères acryliques non polymérisés peuvent être solubles dans le milieu de dispersion considéré, mais le polymère formé avec ces monomères est insoluble dans le milieu de dispersion.
Selon un mode particulier de réalisation de l'invention, le polymère éthylénique greffé est susceptible d'être obtenu par polymérisation radicalaire dans un milieu organique de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide (meth)acrylique, l'acide méthacrylique et les (méth)acrylates d'alkyle de formule (X) définie ci-après, et leurs sels, pour former ledit squelette insoluble ; et
- et d'au moins un macromonomère siliconé comportant un groupe terminal polymérisable, tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'isopropyle, et leurs mélanges.
On peut citer tout particulièrement l'acrylate de méthyle, le méthacrylate de méthyle, le méthacrylate d'éthyle.
Les monomères acryliques additionnels peuvent être choisis parmi :

- l'acide (méth)acrylique et ses sels,
- les (méth)acrylates de formule (X) et leurs sels :

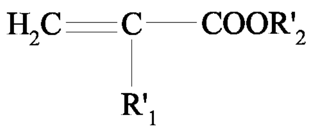

(X)

dans laquelle :

- $R'_1$ désigne un atome d'hydrogène ou un groupe méthyle ;
- $R'_2$ représente :
    - un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi

-OH, les atomes d'halogène (F, Cl, Br, I) et -NR'R" avec R' et R" identiques ou différents choisis parmi les alkyles linéaires ou ramifiés en $C_1$-$C_3$ ;

- un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;
- et leurs mélanges.

A titre d'exemples de $R'_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.

On peut citer tout particulièrement l'acide acrylique et l'acide méthylacrylique.

***b)** Macromonomères*

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryloxy), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure ou égale à 200, de préférence supérieure ou égale à 300, préférentiellement supérieure ou égale à 500, et plus préférentiellement supérieure à 600.

De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8 à C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth)acrylate.

De tels macromonomères sont notamment décrits dans les brevets EP895467 et EP96459 et dans l'article Gillman K.F., Polymer Letters, Vol 5, page 477-481 (1967).

On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène.

De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination KRATON LIQUID L-1253 par KRATON POLYMERS.

Comme macromonomères siliconés, on peut en particulier citer les polydiméthylsiloxanes à groupement terminal mono (méth)acrylate, et notamment ceux de formule (XI) suivante :

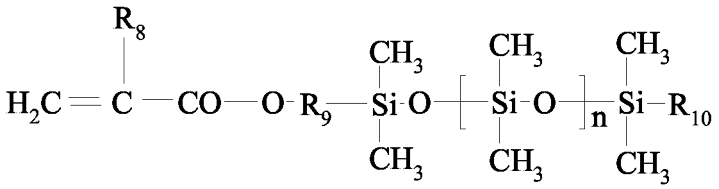

(XI)

dans laquelle :

- $R_8$ désigne un atome d'hydrogène ou un groupement méthyle ;
- $R_9$ désigne un groupe hydrocarboné divalent ayant de 1 à 10 atomes de carbone et contient éventuellement une ou deux liaisons éther -O- ;
- R10 désigne un groupe alkyl ayant de 1 à 10 atomes de carbone, notamment ayant de 2 à 8 atomes de carbone ; et
- n désigne un nombre entier allant de 1 à 300, de préférence allant de 3 à 200, et préférentiellement allant de 5 à 100.

Comme macromonomères siliconés, on peut utiliser les monométhacryloxypropyl polydiméthylsiloxanes tels que ceux commercialisés sous la dénomination PS560-K6 par la société UNITED CHEMICAL TECHNOLOGIES INC. (UCT) ou sous la dénomination MCR-M17 par la société GELEST INC.

Plus particulièrement, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Comme polymère éthylénique greffé particulièrement avantageux dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment KRATON L-1253), en particulier dans un solvant choisi parmi l'isododécane, l'isononanoate d'isononyle, l'octyldodécanol , le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment KRATON L-1253), en particulier dans l'isododécane ;
- des monomères acrylate de méthyle / méthacrylate de méthyle et du macromonomère polyéthylène/polybutylène à groupement terminal méthacrylate (notamment KRATON L-1253), en particulier dans l'isododécane ;
- des monomères acrylate de méthyle / acide acrylique et du macromonomère polyéthylène/polybutylène à groupement terminal méthacrylate (notamment KRATON L-1253), en particulier dans l'isododécane ;
- des monomères acrylate de méthyle / méthacrylate de diméthylaminoéthyle et du macromonomère polyéthylène/polybutylène à groupement terminal méthacrylate (notamment KRATON L-1253), en particulier dans l'isododécane ;
- des monomères acrylate de méthyle / méthacrylate de 2-hydroxyéthyle et du macromonomère polyéthylène/polybutylène à groupement terminal méthacrylate (notamment KRATON L-1253), en particulier dans l'isododécane.

Comme polymère acrylique greffé particulièrement envisagé dispersé dans une phase grasse liquide siliconée, on peut utiliser ceux obtenus par polymérisation :

- de l'acrylate de méthyle et du macromonomère monométhacrylolxypropylpolydiméthylsiloxane ayant un poids moléculaire moyen en poids allant de 800 à 6000, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone.

En particulier, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules

résultant des caractéristiques du polymère greffé ont la particularité de ne pas s'agglomérer dans ledit milieu et donc de s'autostabiliser et de former une dispersion de particules de polymère particulièrement stable.

En particulier, les polymères éthyléniques greffés de la dispersion peuvent former des particules nanométriques, de taille moyenne allant de 10 à 400 nm, de préférence de 20 à 200 nm.

Du fait de cette taille très faible, les particules de polymère greffé en dispersion sont particulièrement stables et donc peu susceptibles de former des agglomérats.

La dispersion de polymère greffé peut donc être une dispersion stable et ne forme pas de sédiments, lorsqu'elle est placée pendant une durée prolongée (par exemple 24 heures) à température ambiante (25 °C).

En particulier, la dispersion de particules de polymère greffé présente un taux de matière sèche (ou extrait sec) en polymère pouvant aller de 40 % à 70 % en poids de matière sèche, notamment allant de 45 % à 65 % en poids.

*c) Procédé* ***d'obtention***

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.

Comme indiqué précédemment, le milieu organique liquide de dispersion peut être identique ou différent du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie du milieu organique de polymérisation, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.

Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.

Le polymère greffé peut être présent dans la composition selon l'invention en une teneur en matière sèche (ou matière active) allant de 1 à 70 % en poids par rapport au poids total de la composition, mieux de 5 à 60% en poids, de préférence allant de 6 à 45% et mieux allant de 8 à 40% en poids.

Dans un mode de réalisation, le polymère filmogène est un polymère organique filmogène soluble dans la phase grasse liquide de la composition, notamment dans la ou les huiles de la composition.

Dans ce cas, on parle de polymère liposoluble. Le polymère liposoluble peut être d'un type chimique quelconque et peut être notamment choisi parmi :

a) **les homopolymères et les copolymères liposolubles et amorphes** des oléfines, des cyclooléfmes, du butadiène, de l'isoprène, du styrène, des éthers, des esters ou amides vinyliques, des esters ou amides de l'acide (méth)acrylique contenant un groupement alkyle en $C_{4-50}$ linéaire, ramifié ou cyclique, et en particulier amorphes. Les homopolymères et les copolymères liposolubles préférés sont obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci. On citera, par exemple, le copolymère d'acrylate d'alkyle/acrylate de cycloalkyle commercialisé par PHOENIX CHEM. sous la dénomination GIOVAREZ AC-5099 ML, et les copolymères de vinylpyrrolidone, tels que les copolymères d'un alkène en $C_2$ à $C_{30}$, tel qu'en $C_3$ à $C_{22}$, et des associations de ceux-ci, peuvent être utilisés. Comme exemples de copolymères de VP pouvant être utilisés dans l'invention, on peut citer le copolymère de VP/laurate de vinyle, de VP/stéarate de vinyle, la polyvinylpyrrolidone (PVP) butylée, de VP/hexadécène, de VP/triacontène ou de VP/acide acrylique/méthacrylate de lauryle.

Comme copolymères liposolubles particuliers, on peut citer :

- i) les polymères de silicone-acrylique greffés ayant un squelette siliconé, des greffons acryliques ou ayant un squelette acrylique, les greffons de silicone tels que le produit commercialisé sous la dénomination SA 70.5 par 3M et décrit dans les brevets US 5 725 882, US 5 209 924, US 4 972 037, US 4 981 903, US 4 981 902, US 5 468 477, et dans les brevets US 5 219 560 et EP 0 388 582,
- ii) les polymères liposolubles portant des groupements fluorés appartenant à l'une des classes décrites dans le texte ci-dessus, en particulier FOMBLIN ceux décrits dans le brevet US 5 948 393, les copolymères de (méth)acrylate d'alkyle/(méth)acrylate de perfluoroalkyle décrits dans les brevets EP 0 815 836 et US 5 849 318,
- iii) les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.

Dans un mode de réalisation, le polymère filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/ propylène (EP), un bloc butadiène (B), un bloc isoprène (I), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination « LUVITOL HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « KRATON » par SHELL CHEMICAL CO. ou GELLED PERMETHYL 99A par PENRECO, peuvent être utilisés.

On peut citer par exemple le KRATON G1650 (SEBS), le KRATON G1651 (SEBS), le KRATON G1652 (SEBS), le KRATON G1657X (SEBS), le KRATON G1701X (SEP), le KRATON G1702X (SEP), le KRATON G1726X (SEB), le KRATON D-1101 (SBS), le KRATON D-1102 (SBS), le KRATON D-1107 (SIS), le GELLED PERMETHYL 99A-750, le GELLED PERMETHYL 99A-753-58 (mélange de polymère bloc en étoile et de polymère tribloc), le GELLED PERMETHYL 99A-753-59 (mélange de polymère bloc en étoile et de polymère tribloc), le VERSAGEL 5970 et le VERSAGEL 5960 de chez PENRECO (mélange de polymère en étoile et de polymère tribloc dans l'isododécane).

Des copolymères de styrène-méthacrylate peuvent également être utilisés tels que les polymère commercialisés sous les références OS 129880, OS 129881 et OS 84383 de chez LUBRIZOL (copolymère de styrène-méthacrylate).

Dans un mode de réalisation, le polymère filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de

polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et en particulier de 4.000 à 200.000.

Comme exemples de polymères liposolubles pouvant être utilisés dans l'invention, on peut citer les polyalkylènes, les copolymères d'alcènes en $C_2$-$C_{20}$, en particulier le polybutène.

b) **les polycondensats amorphes et liposolubles,** en particulier ne comprenant pas de groupements donneurs d'interactions hydrogène, en particulier les polyesters aliphatiques ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, tel que défini dans la demande de brevet FR 0 113 920, et

c) **les polysaccharides amorphes et liposolubles** comprenant des chaînes latérales alkyl (éther ou ester), en particulier les alkylcelluloses ayant un radical alkyle en $C_1$ à $C_8$ saturé ou insaturé, linéaire ou ramifié, tel que l'éthylcellulose et la propylcellulose.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "KETJENTFLEX MS80" de la société AKZO ou "SANTOLITE MHP", "SANTOLITE MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

d) les résines de silicone, généralement solubles ou gonflables dans les huiles de silicone. Ces résines sont des polymères de polyorganosiloxanes réticulés. Par le terme « résine », on entend une structure tridimensionnelle.

Dans un mode de réalisation, la résine de silicone est choisie parmi les silsesquioxanes et les siloxysilicates.

Dans un mode de réalisation, la résine de silicone est choisie parmi les siloxysilicates, tels que les triméthylsiloxysilicates, qui sont représentés par la formule suivante :

$$[R_3SiO_{1/2}]_x\text{-}(SiO_{4/2})_y \text{ (motifs M et Q),}$$

dans laquelle x et y peuvent avoir des valeurs allant de 50 à 80, et R représente un alkyle, tel qu'un méthyle ou un alkyle de deux atomes de carbone ou plus.

Le rapport des motifs M aux motifs Q peut être, par exemple, d'environ 0,7:1. La résine de silicone filmogène peut être choisie, par exemple, parmi les résines WACKER 803 et 804, disponibles auprès de WACKER SILICONE CORPORATION, et G.E. 1 170-002, disponible auprès de GENERAL ELECTRIC.

Dans un autre mode de réalisation, la résine de silicone est choisie parmi les silsesquioxanes qui comprennent des motifs T :

$$[RSiO_{3/2}]_t \text{ (motifs T),}$$

dans laquelle t a une valeur pouvant aller jusqu'à plusieurs milliers et R représente un alkyle, tel qu'un méthyle ou un alkyle de deux atomes de carbone ou plus. Dans un mode de réalisation, le silsesquioxane est choisi parmi les polyméthylsilsesquioxanes, qui sont les silsesquioxanes tel que R est un groupement méthyle.

Les polyméthylsilsesquioxanes peuvent comprendre, par exemple, moins de environ 500 motifs T, préférablement d'environ 50 à environ 500 motifs T.

Tous les polyméthylsilsesquioxanes ne sont pas filmogènes. Par exemple, les polyméthylsilsesquioxanes tels que TOSPEARL™ de chez TOSHIBA ou KMP590 de chez SHIN-ETSU sont très insolubles dans les huiles, et sont de ce fait des agents filmogènes inefficaces. La masse moléculaire de ces polyméthylsilsesquioxanes est difficile à déterminer; ils contiennent généralement un millier ou plus d'un millier de motifs T.

Un exemple d'un polyméthylsilsesquioxane pouvant être utilisé selon l'invention est BELSIL PMS MK (également appelé résine MK), disponible auprès de WACKER CHEMIE. Le polyméthylsilsesquioxane est un polymère constitué principalement de motifs répétitifs $CH_3SiO_{3/2}$ (motifs T) et pouvant également contenir jusqu'à environ 1% (en poids ou en moles) de $(CH_3)_2SiO_{2/2}$ (motifs D).

Les polyméthylsilsesquioxanes convenables pour une utilisation dans la présente invention comprennent KR-220L, disponible auprès de SHIN-ETSU. La structure de KR-220L est constituée essentiellement de motifs de silicone T ($CH_3SiO_{3/2}$) avec des motifs terminaux Si-OH ou silanol. Il n'y a aucun motif D.

Le polyméthylsilsesquioxane KR-242A a une structure ayant environ 98% de motifs méthyle T et environ 2% de motifs diméthyle D, avec des motifs terminaux Si-OH ou silanol, et du KR-251 qui a une structure ayant environ 88% de motifs méthyle T et environ 12% de motifs diméthyle D, avec des motifs terminaux Si-OH ou silanol ; tous les deux sont disponibles auprès de SHIN-ETSU.

Dans un mode de réalisation de l'invention, la résine de silicone est soluble ou dispersable dans les huiles silicones ou des liquides organiques volatils. Dans un mode de réalisation, la résine de silicone est solide à 25°C.

Dans un mode de réalisation, la résine de silicone peut avoir une masse moléculaire allant de 1000 à 10 000 grammes/mole. Dans un mode de réalisation, la résine est présente dans la composition en une quantité allant de 0,5% à 20% en poids par rapport au poids total de la composition, préférablement en une quantité de 1% à 10%.

Dans un mode de réalisation de l'invention, la résine de silicone est choisie parmi les associations de motifs M, D, T et Q, contenant au moins deux motifs choisis parmi M, D, T et Q satisfaisant la relation $R_nSiO_{(4-n)}$, dans laquelle n a une valeur allant de 1,0 à 1,50. Certaines résines de ce type sont décrites dans US-A-6 074 654.

Dans un autre mode de réalisation, la résine de silicone filmogène est un copolymère, dans lequel au moins un motif du copolymère est choisi parmi les motifs de silicone M, D, T et Q, et dans lequel au moins un motif supplémentaire du copolymère est choisi parmi les esters. La résine de silicone filmogène peut être choisie, par exemple, parmi les diisostéaroyltriméthylolpropane siloxysilicates, tels que SF 1 318, disponible auprès de GE SILICONES.

e) **Les copolymères polyamide-silicone** du type polyorganosiloxane tels que ceux décrits dans les documents US-A-5 874 069, US-A-5,919,441, US-A-6,051,216 et US-A-5,981,680.

Selon l'invention, ces polymères siliconés peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Les polymères comportant deux groupes capables d'établir des interactions hydrogène dans la chaîne du polymère peuvent être des polymères comprenant au moins un motif répondant à la formule (XXII) :

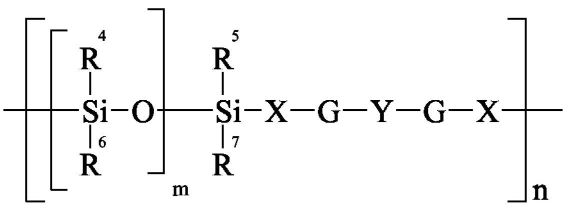

(XXII)

dans laquelle :

1) $R^4$, $R^5$, $R^6$ et $R^7$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyles en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,

3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou

4) Y représente un groupe répondant à la formule (XXIII) :

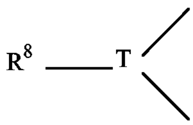
(XXIII)

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

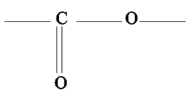 ; 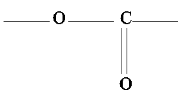 ; 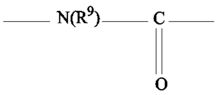 ;

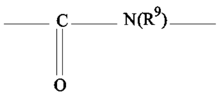 ; 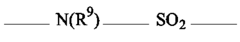 ; 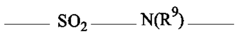 ;

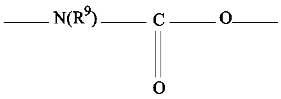 ; 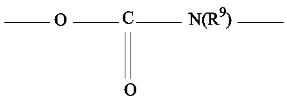 ; 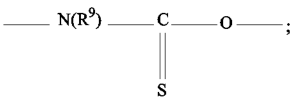 ;

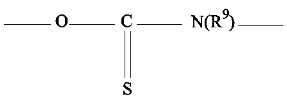 ; 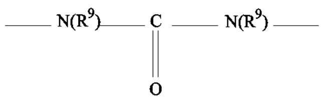 ,

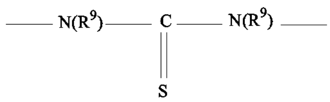 ,

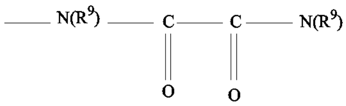 ; 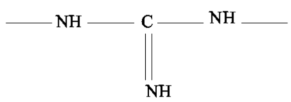 ; et

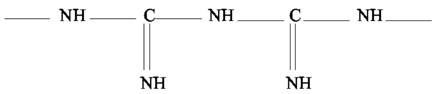

où $R^9$ représente un atome d'hydrogène ou un groupe alkyle, linéaire ou ramifié, en $C_1$ à $C_{20}$, à condition qu'au moins 50% des $R^9$ du polymère représente un atome d'hydrogène et qu'au moins deux des groupes G du polymère soient un autre groupe que :

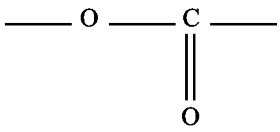 et 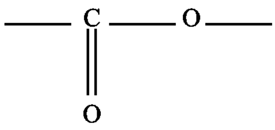 ;

6) n est un nombre entier allant de 2 à 500, en particulier de 2 à 200, et m est un nombre entier allant de 1 à 1000, en particulier de 1 à 700 et mieux encore de 6 à 200.

Selon l'invention, 80 % des $R^4$, $R^5$, $R^6$ et $R^7$, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. En particulier, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule (XXIV) :

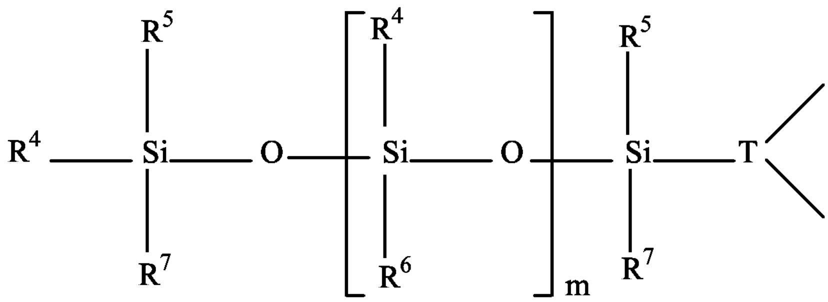

(XXIV)

dans laquelle $R^4$, $R^5$, $R^6$, $R^7$, T et m sont tels que définis ci-dessus, et
h) les chaînes polyorganosiloxanes de formule (XXV) :

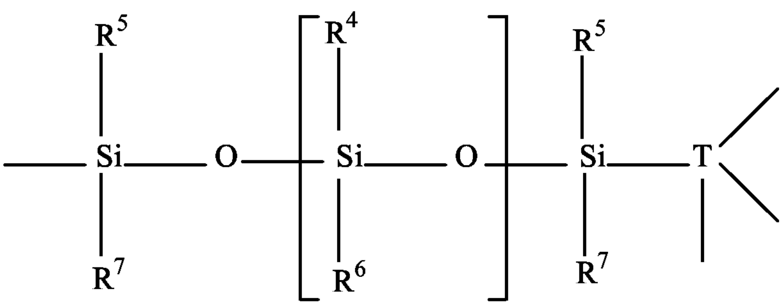

(XXV)

Les polyorganosiloxanes de la seconde famille peuvent être des polymères comprenant au moins un motif répondant à la formule (XXVI) :

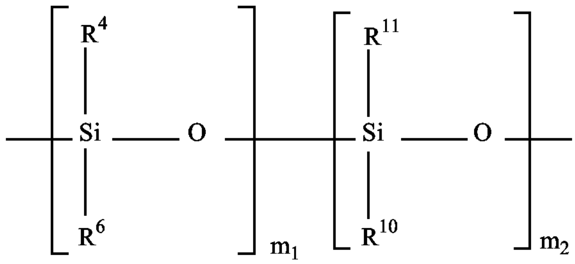

(XXVI)

dans laquelle :

- $R^4$ et $R^6$, identiques ou différents, sont tels que définis ci-dessus pour la formule (XXII),
- $R^{10}$ représente un groupe tel que défini ci-dessus pour $R^4$ et $R^6$, ou représente le groupe de formule $-X-G-R^{12}$ dans laquelle X et G sont tels que définis ci-dessus pour la formule (XXII) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$,
- $R^{11}$ représente le groupe de formule $-X-G-R^9$ dans laquelle X, G et $R^{12}$ sont tels que définis ci-dessus,
- $m_1$ est un nombre entier allant de 1 à 998, et
- $m_2$ est un nombre entier allant de 2 à 500.

Selon l'invention, le polymère utilisé, peut être un homopolymère, c'est-à-dire un polymère comportant plusieurs motifs identiques, en particulier des motifs de formule (XXII) ou de formule (XXVI).

Selon l'invention, on peut aussi utiliser un polymère constitué par un copolymère comportant plusieurs motifs de formule (XXII) différents, c'est-à-dire un polymère dans lequel l'un au moins des $R^4$, $R^5$, $R^6$, $R^7$, X, G, Y, m et n est différent dans l'un des motifs. Le copolymère peut être aussi formé de plusieurs motifs de formule (XXVI), dans lequel l'un au moins des $R^4$, $R^6$, $R^{10}$, $R^{11}$, $m_1$ et $m_2$ est différent dans l'un au moins des motifs.

On peut encore utiliser un copolymère comportant au moins un motif de formule (XXII) et au moins un motif de formule (XXVI), les motifs de formule (XXII) et les motifs de formule (XXVI) pouvant être identiques ou différents les uns des autres.

Selon une variante, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des copolymères blocs, des copolymères séquencés ou des copolymères greffés.

***f)*** Les polymères éthyléniques **séquencés** linéaires

La composition selon l'invention peut contenir, à titre d'agent filmogène un polymère éthylénique séquencé linéaire, appelé par la suite "polymère séquencé", de structure particulière telle que décrite ci-après.

Par polymère "séquencé", on entend un polymère comprenant au moins 2 séquences distinctes, de préférence au moins 3 séquences distinctes.

Le polymère est un polymère à structure linéaire. Par opposition, un polymère de structure non linéaire est, par exemple, un polymère à structure ramifiée, en étoile, greffée, ou autre.

Avantageusement, le polymère séquencé peut être exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène.

De manière particulière, le polymère séquencé comprend au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Par "au moins" une séquence, on entend une ou plusieurs séquences.

La séquence intermédiaire est une séquence comprenant au moins un monomère constitutif de la première séquence et au moins un monomère constitutif de la deuxième séquence du polymère permet de "compatibiliser" ces séquences.

On précise que dans ce qui précède et ce qui suit les termes "première" et "deuxième" séquences ne conditionnent nullement l'ordre desdites séquences (ou blocs) dans la structure du polymère séquencé.
Avantageusement, les première et deuxième séquences et du polymère séquencé sont incompatibles l'une avec l'autre.
Par "séquences incompatibles l'une avec l'autre", on entend que le mélange formé du polymère correspondant à la première séquence et du polymère correspondant à la deuxième séquence, n'est pas miscible dans le liquide organique majoritaire en poids du la phase grasse liquide, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

- i) lesdits polymères sont présents dans le mélange en une teneur telle que le rapport pondéral respectif va de 10/90 à 90/10, et que
- ii) chacun des polymères correspondant au première et seconde séquences a une masse moléculaire moyenne (en poids ou en nombre) égale à celle du polymère séquencé +/- 15 %.

Dans le cas où la composition comporte une phase grasse liquide comprenant un mélange de liquides organiques, et dans l'hypothèse de deux ou plusieurs liquides organiques présents en proportions massiques identiques, ledit mélange de polymères est non miscible dans au moins l'un d'entre eux.
Bien entendu, dans le cas où la phase grasse liquide comprend un unique liquide organique, ce dernier est le liquide organique majoritaire.
De façon particulière, le polymère séquencé ne comprend pas d'atomes de silicium dans son squelette. Par "squelette", on entend la chaîne principale du polymère, par opposition aux chaînes latérales pendantes.
En particulier, le polymère séquencé n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).
En particulier, le polymère séquencé n'est pas un élastomère.
Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.
De manière plus spécifique, par "polymère non élastomère" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.

***i) Test** de **recouvrance***

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.
On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.
On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après :

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.

La recouvrance retardée en % ($R_{2h}$) est donnée par la formule ci-après:

$$R_{2h}= (\varepsilon_{max} - \varepsilon_{2h})/\varepsilon_{max}) \text{ x } 100$$

A titre purement indicatif, un polymère selon un mode de réalisation de invention possède une recouvrance instantanée $R_i$ de 10% et une recouvrance retardée $R_{2h}$ de 30%.

Avantageusement, le polymère séquencé a un indice de polydispersité I supérieur à 2, par exemple allant de 2 à 9, en particulier supérieur ou égal à 2,5, par exemple allant de 2,5 à 8, et plus particulièrement supérieur ou égal à 2,8 et notamment, allant de 2,8 à 6.

L'indice de polydispersité I du polymère séquencé est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère séquencé est en particulier inférieure ou égale à 300 000, elle va par exemple de 35 000 à 200 000, et plus particulièrement de 45 000 à 150 000.

La masse moyenne en nombre (Mn) du polymère séquencé est en particulier inférieure ou égale à 70 000, elle va par exemple de 10 000 à 60 000, et plus particulièrement de 12 000 à 50 000.

Chaque séquence ou bloc du polymère séquencé est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Avantageusement, la séquence intermédiaire comprenant au moins un monomère constitutif de la première séquence et au moins un monomère constitutif de la deuxième séquence du polymère séquencé est un polymère statistique.

En particulier, la séquence intermédiaire est issue essentiellement de monomères constitutifs de la première séquence et de la deuxième séquence.

Par "essentiellement", on entend au moins à 85%, en particulier au moins à 90%, en particulier à 95% et encore plus particulièrement à 100%.

Avantageusement, la séquence intermédiaire a une température de transition vitreuse Tg comprise entre les températures de transition vitreuse des première et deuxième séquences.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg= \sum_{i} (\acute{\omega}_i/Tg_i) \text{ ,}$$

$\acute{\omega}_i$ étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse de la première et de la deuxième séquences est généralement supérieur à 10 °C, en particulier supérieur à 20 °C, et en particulier supérieur à 30 °C.

***ii) <u>Séquences</u> du <u>polymère</u>***

En particulier, la première séquence du polymère séquencé peut être choisie parmi :

a) une séquence ayant une Tg supérieure ou égale à 40 °C,
b) une séquence ayant une Tg inférieure ou égale à 20 °C,
c) une séquence ayant une Tg comprise entre 20 et 40 °C,

et la deuxième séquence choisie dans une catégorie a), b) ou c) différente de la première séquence.

On entend désigner dans la présente invention, par l'expression "compris entre ... et ...", un intervalle de valeurs dont les bornes mentionnées sont exclues, et "de ... à ..." et "allant de ... à ...", un intervalle de valeurs dont les bornes sont inclues.

a) **<u>Séquence avant une Tg supérieure ou égale à 40 °C</u>**

La séquence ayant une Tg supérieure ou égale à 40 °C a par exemple une Tg allant de 40 à 150 °C, en particulier supérieure ou égale à 50 °C, allant par exemple de 50 °C à 120 °C, et en particulier supérieure ou égale à 60 °C, allant par exemple de 60 °C à 120 °C.

La séquence ayant une Tg supérieure ou égale à 40°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse supérieures ou égales à 40 °C. Cette première séquence peut être un homopolymère, constitué par un seul type de monomère (dont la Tg de l'homopolymère correspondant est supérieure ou égale à 40 °C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40 °C, par exemple une Tg allant de 40 à 150 °C, en particulier supérieure ou égale à 50 °C, allant par exemple de 50 °C à 120 °C, et en particulier supérieure ou égale à 60 °C, allant par exemple de 60 °C à 120 °C, et
- des monomères qui sont tel(s) que les homopolymères préparés à partir de ces monomères ont des Tg inférieures à 40 °C, choisis parmi les monomères ayant une Tg comprise entre 20 à 40 °C et/ou les monomères ayant une Tg inférieure ou égale à 20 °C, par exemple une Tg allant de -100 à 20 °C, en particulier inférieure à 15 °C, notamment allant de - 80 °C à 15 °C et en particulier inférieur à 10 °C, par exemple allant de -50 °C à 0 °C à, tels que décrits plus loin.

Les monomères dont les homopolymères ont une température de transition vitreuse supérieure ou égale à 40 °C sont, de préférence, choisis parmi les monomères suivants, appelés aussi monomères principaux :

- les méthacrylates de formule (XII) :

$$CH_2 = C(CH_3)\text{-}COOR_1 \qquad \text{(XII)}$$

  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule (XIII) :

$$CH_2 = CH\text{-}COOR_2 \qquad \text{(XIII)}$$

  dans laquelle $R_2$ représente un groupe cycloalkyle en $C_4$ à $C_{12}$ tel que l'acrylate d'isobornyle ou un groupe tertio butyle,
- les (méth)acrylamides de formule (XIV) :

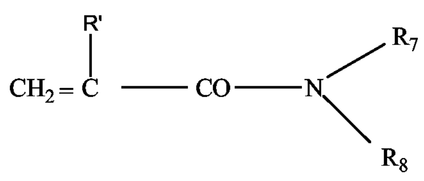

(XIV)

où :

- $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et
- R' désigne H ou méthyle,

- et leurs mélanges.

Comme exemple de monomères, on peut citer le N-butylacrylamide, le N-t-butylacrylamide, le N-isopropylacrylamide, le N,N-diméthylacrylamide et le N,N-dibutylacrylamide.
Des monomères principaux particulièrement avantageux sont le méthacrylate de méthyle, le (méth)acrylate d'isobutyle, le (méth)acrylate d'isobornyle et leurs mélanges.

b) **Séquence avant une Tg inférieure ou égale à 20 °C**

La séquence ayant une Tg inférieure ou égale à 20 °C a par exemple une Tg allant de -100 à 20 °C, de préférence inférieure ou égale à 15 °C, notamment allant de -80 °C à 15 °C et mieux inférieure ou égale à 10 °C, par exemple allant de -50 °C à 0 °C.
La séquence ayant une Tg inférieure ou égale à 20°C peut être un homopolymère ou un copolymère.
Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).
Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20 °C, par exemple une Tg allant de -100 °C à 20 °C, en particulier inférieure à 15 °C, notamment allant de -80 °C à 15 °C et en particulier inférieur à 10 °C, par exemple allant de -50 °C à 0 °C et
- un ou plusieurs monomères dont l'homopolymère correspondant a une Tg supérieure à 20 °C, tels que les monomères ayant une Tg supérieure ou égale à 40 °C, par exemple une Tg allant de 40 à 150 °C, en particulier supérieure ou égale à 50 °C, allant par exemple de 50 °C à 120 °C, et en particulier supérieure ou égale à 60 °C, allant par exemple de 60 °C à 120 °C et /ou les monomère ayant une Tg comprise entre 20 et 40 °C, tels que décrits plus haut.

En particulier, la séquence ayant une Tg inférieure ou égale à 20 °C est un homopolymère.
Les monomères dont l'homopolymère a une Tg inférieure ou égale à 20 °C sont, de préférence, choisis parmi les monomères suivants, ou monomère principaux :

- les acrylates de formule (XV) :

$$CH_2 = CHCOOR_3 \qquad (XV)$$

  $R_3$ représentant un groupe alkyle non substitué en $C_1$ à $C_{12}$, linéaire ou ramifié, dans lequel se trouve(nt) éventuellement intercalé(s) un ou plusieurs hétéroatomes choisis parmi O, N, S,
- les méthacrylates de formule (XVI) :

$$CH_2 = C(CH_3)\text{-}COOR_4 \qquad (XVI)$$

  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule (XVII) :

$$R_5\text{-CO-O-CH} = CH_2 \qquad (XVII)$$

  où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

Les monomères principaux particulièrement préférés pour la séquence ayant une Tg inférieure ou égale à 20 °C sont les acrylates d'alkyles dont la chaîne alkyle comprend de 1 à 10 atomes de carbone, tels que l'acrylate de méthyle, l'acrylate d'isobutyle, l'acrylate d'éthyl-2 hexyle et leurs mélanges.

c) **Séquence avant une Tg comprise entre 20 et 40°C**

La séquence qui a une Tg comprise entre 20 et 40 °C peut être un homopolymère ou un copolymère.

Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40 °C. Cette première séquence peut être un homopolymère, constitué par un seul type de monomère (dont la Tg de l'homopolymère correspondant va de 20 °C à 40 °C).

Les monomères dont l'homopolymère a une température de transition vitreuse comprise entre 20 et 40 °C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40 °C.

Avantageusement, la séquence ayant une Tg comprise entre 20 et 40 °C est un copolymère issu en totalité ou en partie :

- de monomères principaux dont l'homopolymère correspondant a une Tg supérieure ou égale à 40 °C, par exemple une Tg allant de 40 °C à 150 °C, en particulier supérieure ou égale à 50 °C, allant par exemple de 50 à 120 °C, et mieux supérieure ou égale à 60 °C, allant par exemple de 60 °C à 120 °C, tels que décrits plus haut, et/ou
- de monomères principaux dont l'homopolymère correspondant a une Tg inférieure ou égale à 20 °C, par exemple une Tg allant de -100 à 20 °C, en particulier inférieure ou égale à 15 °C, notamment allant de -80 °C à 15 °C et en particulier inférieure ou égale à 10 °C, par exemple allant de -50 °C à 0 °C, tels que décrits plus haut,
  lesdits monomères étant choisis de telle sorte que la Tg du copolymère formant la première séquence est comprise entre 20 et 40 °C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.

Plus particulièrement, la proportion de la deuxième séquence ayant une Tg inférieure ou égale à 20 °C va de 10 à 85 % en poids du polymère, mieux de 20 à 70 % et encore mieux de 20 à 50 %.

Chacune des séquences peut néanmoins contenir en proportion minoritaire au moins un monomère constitutif de l'autre séquence.

Ainsi la première séquence peut contenir au moins un monomère constitutif de la deuxième séquence et inversement.

Chacune des première et/ou deuxième séquence du polymère séquencé peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

***iii) Monomère additionnel***

Ce monomère additionnel est par exemple choisi parmi :

- les monomères hydrophiles tels que :
  - les monomères à insaturation(s) éthylénique(s) comprenant au moins une fonction acide carboxylique ou sulfonique comme par exemple :
    - l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
  - les monomères à insaturation(s) éthylénique(s) comprenant au moins une fonction amine tertiaire comme :
    - la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
    - les méthacrylates de formule (XVIII) :

$$CH_2 = C(CH_3)\text{-}COOR_6 \quad (XVIII)$$

dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,

- les méthacrylates de formule (XIX) :

$$CH_2 = C(CH_3)\text{-}COOR_9 \quad (XIX)$$

dans laquelle $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule (XX) :

$$CH_2 = CHCOOR_{10} \quad (XX)$$

dans laquelle $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylène comprenant de 5 à 30 motifs d'oxyde d'éthylène.

- les monomères à insaturation éthylénique comprenant un ou plusieurs atomes de silicium tels que le méthacryloxypropyl triméthoxy silane, le méthacryloxypropyl tris ( triméthylsiloxy ) silane,
- et leurs mélanges.

Des monomères additionnels particulièrement préférés sont l'acide acrylique, l'acide méthacrylique, le méthacrylate de trifluoroéthyle et leurs mélanges.

Selon un mode particulier de réalisation, le polymère séquencé est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

En particulier, chacune des première et deuxième séquences comprend au moins un monomère choisi parmi les esters d'acide (méth)acrylique, et éventuellement au moins un monomère choisi parmi l'acide (méth)acrylique, et leurs mélanges.

Avantageusement, chacune des première et deuxième séquences du polymère séquencé est issue en totalité d'au moins un monomère choisi parmi l'acide acrylique, les esters d'acide (méth)acrylique, et éventuellement d'au moins un monomère choisi parmi l'acide (méth)acrylique, et leurs mélanges.

***iv)** <u>Procédé **d'obtention**</u>*

Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120 °C),
- une fois cette température atteinte, les monomères constitutifs de la première séquence sont introduits en présence d'une partie de l'initiateur de polymérisation,
- au bout d'un temps T correspondant à un taux de conversion maximum de 90 %, les monomères constitutifs de la deuxième séquence et l'autre partie de l'initiateur sont introduits,
- on laisse réagir le mélange pendant un temps T' (allant de 3 à 6 h) au bout duquel le mélange est ramené à

température ambiante,

- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De manière particulière, le solvant de polymérisation est un mélange acétate de butyle et isopropanol ou l'isododécane.

Selon un mode particulier de réalisation, le polymère séquencé comprend une première séquence ayant une Tg supérieure ou égale à 40 °C, telle que décrite plus haut au a) et une deuxième séquence ayant une Tg inférieure ou égale à 20 °C, telle que décrite plus haut au b).

En particulier, la première séquence ayant une Tg supérieure ou égale à 40 °C est un copolymère issu de monomères qui sont tels que homopolymère préparé à partir de ces monomères a une température de transition vitreuse supérieure ou égale à 40 °C, tels que les monomère décrits plus haut.

Avantageusement, la deuxième séquence ayant une Tg inférieure ou égale à 20 °C est un homopolymère issu de monomères qui sont tel(s) que l'homopolymère préparé à partir de ces monomères a une température de transition vitreuse inférieure ou égale à 20 °C, tels que les monomères décrits plus haut.

En particulier, la proportion de la séquence ayant une Tg supérieure ou égale à 40 °C va de 20 à 90 % en poids du polymère, mieux de 30 à 80 % et encore mieux de 50 à 70%.

En particulier, la proportion de la séquence ayant une Tg inférieure ou égale à 20 °C va de 5 à 75 % en poids du polymère, de préférence de 15 à 50 % et mieux de 25 à 45 %.

Avantageusement, le polymère séquencé peut comprendre :

- une première séquence de Tg supérieure ou égale à 40 °C, par exemple allant de 85 à 115 °C, qui est un copolymère acrylate d'isobornyle/méthacrylate d'isobutyle,
- une deuxième séquence de Tg inférieure ou égale à 20 °C, par exemple allant de -85 à -55 °C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon un autre mode de réalisation, le polymère séquencé comprend une première séquence ayant une température de transition vitreuse (Tg) comprise entre 20 et 40 °C, conforme aux séquences décrites c) et une deuxième séquence ayant une température de transition vitreuse inférieure ou égale à 20 °C, telle que décrite plus haut au b) ou une température de transition vitreuse supérieure ou égale à 40 °C, telle que décrite ci-dessus.

En particulier, la proportion de la première séquence ayant une Tg comprise entre 20 et 40 °C va de 10 à 85 % en poids du polymère, en particulier de 30 à 80 % et encore mieux de 50 à 70 %.

Lorsque la deuxième séquence est une séquence ayant une Tg supérieure ou égale à 40 °C, elle est en particulier présente en une proportion allant de 10 à 85 % en poids du polymère, en particulier de 20 à 70 % et plus particulièrement de 30 à 70 %.

Lorsque la deuxième séquence est une séquence ayant une Tg inférieure ou égale à 20 °C, elle est en particulier présente en une proportion allant de 10 à 85 % en poids du polymère, en particulier de 20 à 70 % et plus particulièrement de 20 à 50 %.

En particulier, la première séquence ayant une Tg comprise entre 20 et 40 °C est un copolymère issu de monomères qui sont tel(s) que l'homopolymère correspondant a une Tg supérieure ou égale à 40 °C et de monomères qui sont tel(s) que l'homopolymère correspondant a une Tg inférieure ou égale à 20 °C.

Avantageusement, la deuxième séquence ayant une Tg inférieure ou égale à 20 °C ou ayant une Tg supérieure ou égale à 40 °C est un homopolymère.

Selon une première variante, le polymère séquencé comprend :

- une première séquence de Tg comprise entre 20 et 40 °C, par exemple ayant une Tg de 21 à 39 °C, qui est un copolymère comprenant acrylate d'isobornyle/méthacrylate d'isobutyle/acrylate d'éthyl-2 hexyle,
- une deuxième séquence de Tg inférieure ou égale à 20 °C, par exemple allant de -65 à -35 °C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une autre variante, le polymère séquencé peut comprendre :

- une première séquence de Tg supérieure ou égale à 40 °C, par exemple allant de 85 à 115 °C, qui est un copolymère

méthacrylate d'isobornyle/méthacrylate d'isobutyle,

- une deuxième séquence de Tg inférieure ou égale à 20 °C, par exemple allant de -35 à -5 °C, qui est un homopolymère d'acrylate d'isobutyle et
- une séquence intermédiaire qui est un copolymère statistique méthacrylate d'isobornyle/méthacrylate d'isobutyle/acrylate d'isobutyle.

Selon encore une autre variante, le polymère séquencé peut comprendre :

- une première séquence de Tg supérieure ou égale à 40 °C, par exemple allant de 60 à 90 °C, qui est un copolymère acrylate d'isobornyle/méthacrylate d'isobutyle,
- une deuxième séquence de Tg inférieure ou égale à 20 °C, par exemple allant de -35 à -5 °C, qui est un homopolymère d'acrylate d'isobutyle et
- une séquence intermédiaire qui est un copolymère statistique acrylate d'isobornyle/méthacrylate d'isobutyle/acrylate d'isobutyle.

g) **les produits de réaction entre un dérivé de silice et un polydiorganosiloxane portant des groupes terminaux silanol,** tels que décrits dans les brevets US 5 162 410, US 330 747 et US 5 451 610 dont el contenu est incorporé dans la présente demande par référence. De tels produits sont notamment ceux commercialisés sous lé référence Bio-PSA par DOW Corning, par exemple le produit de cette gamme référencé 7-4405.

Selon l'invention le polymère filmogène peut être un solide insoluble dans la phase grasse de la composition à une température ambiante, par exemple, d'environ 25 °C. Le polymère est également insoluble dans la phase grasse à sa température de ramollissement, à l'inverse d'une cire même d'origine polymérique qui est elle soluble dans la phase organique liquide (ou phase grasse) à sa température de fusion. En ce sens, le polymère n'est pas une cire.

***1) Polymères***

La composition selon l'invention comprend avantageusement au moins une dispersion stable de particules de polymère essentiellement sphériques d'un ou plusieurs polymères, dans une phase grasse physiologiquement acceptable.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1$\mu$m.

En particulier, les particules de polymères en dispersion sont insolubles dans les alcools hydrosolubles tels que, par exemple, l'éthanol.

Les polymères en dispersion utilisables dans la composition de l'invention ont de préférence un poids moléculaire de l'ordre de 2000 à 10 000 000 g/mol, et une Tg de - 100°C à 300°C et mieux de -50° à 100°C, de préférence de -10°C à 50°C.

Il est possible d'utiliser des polymères filmifiables, de préférence ayant une Tg basse, inférieure ou égale à la température de la peau et notamment inférieure ou égale à 40°C.

Parmi les polymères filmogènes, on peut citer des homopolymères ou des copolymères radicalaires, acryliques ou vinyliques, de préférence ayant une Tg inférieure ou égale à 40°C et notamment allant de - 10° à 30°C, utilisés seul ou en mélange.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères acryliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise en particulier l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de

benzyle ou de phényle.

Les esters de l'acide (méth)acrylique qui conviennent particulièrement pour les compositions cosmétiques selon l'invention sont les (méth)acrylates d'alkyle.

Comme polymère radicalaire, on utilise en particulier les copolymères d'acide (méth)acrylique et de (méth)acrylate d'alkyle, notamment d'alkyle en $C_1$-$C_4$. Plus particulièrement, on peut utiliser les acrylates de méthyle éventuellement copolymérisés avec l'acide acrylique.

Comme amides des monomères acides, on peut citer les (méth)acrylamides, et notamment les N-alkyl (méth)acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase grasse peuvent représenter en matière sèche de 5 à 40% du poids de la composition, de préférence de 5 à 35 % et mieux de 8 à 30%.

***2) <u>Stabilisant</u>***

Selon un mode de mise en oeuvre, les particules de polymère en dispersion sont stabilisées en surface par un stabilisant solide à température ambiante. Dans ce cas, la quantité en matière sèche de la dispersion représente la quantité totale de polymère + stabilisant, sachant que la quantité de polymère ne peut être inférieure à 5%.

Les particules de polymère sont en particulier stabilisées en surface grâce à un stabilisant, qui peut être un polymère séquencé, un polymère greffé, et/ou un polymère statistique, seul ou en mélange. La stabilisation peut être effectuée par tout moyen connu, et en particulier par ajout direct du polymère stabilisant lors de la polymérisation.

Le stabilisant peut être également présent dans le mélange avant polymérisation du polymère. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ;

les polymères hydrocarbonés greffés avec une chaîne siliconée.

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de "KRATON" par SHELL CHEMICAL Co ou encore du type polystyrène/copoly(éthylène-butylène) (SEB). En particulier, on peut utiliser le KRATON G1650 (SEBS), le KRATON G1651 (SEBS), le KRATON G1652 (SEBS), le KRATON G1657X (SEBS), le KRATON G1701X (SEP), le KRATON G1702X (SEP), le KRATON G1726X (SEB), le KRATON D-1101 (SBS), le KRATON D-1102 (SBS), le KRATON D-1107 (SIS). Les polymères sont généralement appelés des copolymères de diènes hydrogénés ou non.

On peut aussi utiliser les GELLED PERMETHYL 99A-750, 99A-753-59 et 99A-753-58 (mélange de tribloc et de polymère en étoile), VERSAGEL 5960 de chez PENRECO (tribloc + polymère en étoile) ; OS129880, OS129881 et OS84383 de chez LUBRIZOL (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse du polymère est apolaire, il est avantageux de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le solvant de synthèse ne comprend pas d'huile de silicone, l'agent stabilisant est de préférence choisi dans le groupe constitué par :

a) les copolymères blocs greffés ou séquencés comprenant au moins un bloc de type polyorganosiloxane et au moins un bloc d'un polymère radicalaire ou d'un polyéther ou d'un polyester,

b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,

c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

Le polymère filmogène liposoluble ou en dispersion dans une phase grasse peut également être utilisé en une quantité allant de 0,01% à 20% (en matière active) par rapport au poids total de la composition, tel que par exemple de 1% à 10%, le cas échéant.

**I. <u>Filmogène dispersible dans une phase aqueuse de la composition</u>**

Selon un autre mode de réalisation, le polymère filmogène peut être choisi parmi les dispersions aqueuses de particules polymères, dans le cas où la composition selon l'invention comprend une phase aqueuse.

La dispersion aqueuse comprenant un ou plusieurs polymères filmogènes peut être préparée par l'homme de l'art sur la base de ses connaissances générales, en particulier par une polymérisation en émulsion ou par une mise en dispersion du polymère précédemment formé.

Parmi les polymères filmogènes pouvant être utilisés dans la composition selon la présente invention, on peut citer les polymères synthétiques du type polycondensat ou du type radical, les polymères d'origine naturelle, et des mélanges de ceux-ci.

***1) Polycondensats***

Parmi les polycondensats, on peut également citer les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino), comprenant en outre soit un groupement carboxylate ou acide carboxylique, soit un groupement sulfonate ou acide sulfonique, voire un groupement amine tertiaire neutralisable ou un groupement ammonium quaternaire.

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques. Le glycérol, le pentaérythritol, le sorbitol et le triméthylolpropane peuvent être utilisés comme polyols.

Les polyesteramides peuvent être obtenus de manière analogue aux polyesters, au moyen de la polycondensation de diacides avec des diamines ou des aminoalcools. L'éthylènediamine, l'hexaméthylènediamine, et la méta- ou para-phénylènediamine peuvent être utilisées comme diamine. La monoéthanolamine peut être utilisée comme aminoalcool.

Comme monomère portant un groupement anionique pouvant être utilisé pendant la polycondensation, on peut citer par exemple, l'acide diméthylolpropionique, l'acide trimellitique ou un dérivé tel que l'anhydride trimellitique, le sel de sodium de l'acide 3-sulfopentanediol et le sel de sodium de l'acide 5-sulfo-1,3-benzènedicarboxylique. Les polyesters ayant une chaîne grasse peuvent être obtenus par l'intermédiaire de l'utilisation de diols ayant une chaîne grasse lors

de la polycondensation. Les résines d'époxyester peuvent être obtenues par la polycondensation d'acides gras avec un condensat au niveau des extrémités $\alpha,\omega$-diépoxy.

Les polymères radicalaires peuvent être en particulier les polymères ou les copolymères acryliques et/ou vinyliques. Les polymères à radical anionique sont préférés. Comme monomère portant un groupement anionique pouvant être utilisé lors de la polymérisation radicalaire, on peut citer l'acide acrylique, l'acide méthacrylique, l'acide crotonique, l'anhydride maléique et l'acide 2-acrylamido-2-méthylpropanesulfonique.

Les polymères acryliques peuvent résulter de la copolymérisation de monomères choisis parmi les esters et/ou les amides de l'acide acrylique ou de l'acide méthacrylique. Comme exemples de monomères du type ester, on peut citer le méthacrylate de méthyle, le méthacrylate d'éthyle, le méthacrylate de butyle, le méthacrylate d'isobutyle, le méthacrylate de 2-éthylhexyle et le méthacrylate de lauryle. Comme exemples de monomères du type amide, on peut citer le N-t-butylacrylamide et le N-t-octylacrylamide.

On utilise en particulier les polymères acryliques obtenus par la copolymérisation de monomères à insaturation éthylénique contenant des groupements hydrophiles, préférablement de nature non ionique, tels que l'acrylate d'hydroxyéthyle, l'acrylate de 2-hydroxypropyle, le méthacrylate d'hydroxyéthyle et le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemples d'esters vinyliques, on peut citer l'acétate de vinyle, le néodécanoate de vinyle, le pivalate de vinyle, le benzoate de vinyle et le t-butylbenzoate de vinyle.

On peut également utiliser des copolymères d'acrylique/silicone, voire des copolymères de nitrocellulose/acrylique.

***2) Polymère type radical***

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

Lorsqu'une dispersion aqueuse de particules polymères est utilisée, la teneur en matière sèche de ladite dispersion aqueuse peut être de l'ordre de 3 à 60% en poids, et préférablement de 10 à 50%.

La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.

Comme dispersion aqueuse de polymère filmogène, on peut utiliser les dispersions acryliques vendues sous les dénominations « NEOCRYL XK-90® », «NEOCRYL A-1070® », «NEOCRYL A-1090® », «NEOCRYL BT-62® », « NEOCRYL A-1079® » et « NEOCRYL A-523® » par la société AVECIA-NEORESINS, « DOW LATEX 432® » par la société DOW CHEMICAL, « DAITOSOL 5000 AD® » ou « DAITOSOL 5000 SJ » par la société DAITO KASEY KOGYO; « SYNTRAN 5760 » par la société INTERPOLYMER ou encore les dispersions aqueuses de polyuréthane vendues sous les dénominations «NEOREZ R-981® » et «NEOREZ R-974® » par la société AVECIA-NEORESINS, les « AVALURE UR-405® », « AVALURE UR-410® », « AVALURE UR-425® », « AVALURE UR-450® », « SANCURE 875® », « SANCURE 861® », « SANCURE 878® » et « SANCURE 2060® » par la société GOODRICH, «IMPRANIL 85® » par la société BAYER, «AQUAMERE H-1511® » par la société HYDROMER ; les sulfopolyesters vendus sous le nom de marque « EASTMAN AQ® » par la société EASTMAN CHEMICAL PRODUCTS, les dispersions vinyliques comme le « MEXOMERE PAM », les dispersions aqueuses de polyvinyl acétate comme le « VINYBRAN® » de la société NISSHIN CHEMICAL ou celles commercialisées par la société UNION CARBIDE, les dispersions aqueuses de terpolymère vinyl pyrrolidone, diméthylaminopropyl méthacrylamide et chlorure de lauryldiméthylpropylmethacrylamidoammonium telles que le STYLEZE W-d'ISP, les dispersion aqueuse de polymère hybrides polyuréthane/polyacryliques telles que celles commercialisées sous les références « HYBRIDUR ® » par la société AIR PRODUCTS ou « DUROMER ® » de NATIONAL STARCH, les dispersions type core/shell : par exemple celles commercialisées par la société ATOFINA sous la référence KYNAR (core : fluoré - shell : acrylique) ou encore ceux décrits dans le document US 5 188 899 (core : silice - shell : silicone) et leurs mélanges.

Dans le cas où la composition comprend une phase aqueuse, le polymère filmogène peut être un polymère hydrosoluble. Le polymère hydrosoluble est donc solubilisé dans la phase aqueuse de la composition.

Parmi les polymères filmogènes hydrosolubles, on peut citer les polymères cationiques suivants :

1) les polymères ou copolymères acryliques, tels que les polyacrylates ou les polyméthacrylates ; les copolymères de la famille (1) peuvent contenir en outre un ou plusieurs motifs dérivant de comonomères pouvant être choisis parmi la famille des acrylamides, des méthacrylamides, des diacétoneacrylamides, des acrylamides et méthacrylamides substitués sur l'azote par des alkyles inférieurs, des acides acrylique ou méthacrylique ou des esters de ceux-ci, des vinyllactames tels que la vinylpyrrolidone ou le vinylcaprolactame, des esters vinyliques.

Ainsi, parmi ces copolymères de la famille (1), on peut citer :

- les copolymères d'acrylamide et de méthacrylate de diméthylaminoéthyle quaternisés par le sulfate de diméthyle, ou par un halogénure de diméthyle tel que celui commercialisé sous la dénomination HERCOFLOC par la société HERCULES,
- le copolymère d'acrylamide et de chlorure de méthacryloyloxyéthyltriméthylammonium décrit, par exemple, dans la demande de brevet EP-A-0 809 76 et commercialisé sous la dénomination BINA QUAT P 100 par la société CIBA GEIGY,
- le copolymère d'acrylamide et de méthosulfate de méthacryloyloxyéthyltriméthylammonium commercialisé sous la dénomination RETEN par la société HERCULES,
- les copolymères de vinylpyrrolidone/acrylate ou méthacrylate de dialkylaminoalkyle, quaternisés ou non quaternisés, tels que les produits commercialisés sous la dénomination « GAFQUAT » par la société ISP, tels que par exemple « GAFQUAT 734 » ou « GAFQUAT 755 », ou bien les produits désignés par « COPOLYMER 845, 958 et 937 ». Ces polymères sont décrits en détail dans les brevets français 2 077 143 et 2 393 573,
- les terpolymères de méthacrylate de diméthyl-aminoéthyle/vinylcaprolactame/vinylpyrrolidone tels que le produit commercialisé sous la dénomination GAFFIX VC 713 par la société ISP, et
- le copolymère de vinylpyrrolidone/diméthylaminopropyl-méthacrylamide quaternisé tel que le produit commercialisé sous la dénomination « GAFQUAT HS 100 » par la société ISP.

2) les polysaccharides quaternisés décrits plus particulièrement dans les brevets US 3 589 578 et US 4 031 307, tels que les gommes de guar contenant des groupements trialkylammonium cationiques. De tels produits sont commercialisés en particulier sous les dénominations commerciales JAGUAR C13 S, JAGUAR C 15 et JAGUAR C 17 par la société MEYHALL.
3) les copolymères de vinylpyrrolidone et de vinylimidazole quaternaires ;
4) les chitosanes ou les sels de ceux-ci ;
5) les dérivés de cellulose cationiques, tels que les copolymères de cellulose ou de dérivés de cellulose greffés par un monomère hydrosoluble comprenant un ammonium quaternaire et décrits en particulier dans le brevet US 4 131 576, tels que les hydroalkyl celluloses, comme les hydroxyméthyl, hydroxyéthyl ou hydroxypropyl celluloses greffées en particulier par un sel de méthacryloyloxyéthyltriméthylammonium, de méthacrylamidopropyltriméthylammonium ou de diméthyldiallylammonium. Les produits commercialisés correspondant à cette définition sont plus particulièrement les produits commercialisés sous la dénomination « CELQUAT L 200 » et « CELQUAT H 100 » par la NATIONAL STARCH COMPANY.

Parmi les polymères hydrosolubles filmogènes, on peut citer les polymères amphotères suivants :

1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloroacrylique, et d'un monomère basique dérivé d'un composé vinylique substitué contenant au moins un atome de base tel que plus particulièrement un méthacrylate et acrylate de dialkylamino-alkyle, et un dialkylaminoalkylméthacrylamide et acrylamide. De tels composés sont décrits dans le brevet américain n° 3 836 537,
2) les polymères comprenant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que les esters, ayant des substituants amine primaire, secondaire, tertiaire et quaternaire, d'acides acrylique et méthacrylique, et le produit de la quaternisation du méthacrylate de diméthylaminoéthyle par du sulfate de diméthyle ou de diéthyle.
d) les alkoylpolyaminoamides réticulés dérivés totalement ou en partie de polyaminoamides,

3) les polymères comprenant des motifs zwitterioniques,
4) le polymère dérivé du chitosane,
5) les polymères dérivés de la N-carboxyalkylation du chitosane, tels que le N-carboxyméthyl chitosane ou le N-carboxybutyl chitosane commercialisé sous la dénomination « EVALSAN » par la société JAN DEKKER,
6) les copolymères du ($C_1$-$C_5$)alkylvinyléther/ anhydride maléique partiellement modifié par une semi-amidification par une N,N-dialkylaminoalkylamine, telle que la N,N-diméthylaminopropylamine ou par une semi-estérification par une N,N-dialcanolamine. Ces copolymères peuvent également comporter d'autres comonomères vinyliques tels que le vinylcaprolactame.

Les polymères filmogènes hydrosolubles sont préférablement choisis parmi le groupe constitué par :

- les protéines telles que les protéines d'origine végétale, telles que les protéines de blé ou de soja ; les protéines d'origine animale, telles que la kératine, par exemple les hydrolysats de kératine et les kératines sulfoniques ;
- les polymères anioniques, cationiques, amphotères ou non ioniques de la chitine ou du chitosane ;
- les polymères cellulosiques, tels que l'hydroxyéthyl cellulose, l'hydroxypropyl cellulose, la méthylcellulose, l'éthylhydroxyéthyl cellulose, la carboxyméthyl cellulose, et les dérivés quaternisés de la cellulose ;
- les polymères ou copolymères acryliques tels que les polyacrylates ou les polyméthacrylates ;
- les polymères vinyliques, tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle ;
- les copolymères de la vinylpyrrolidone et du caprolactame ; les alcools polyvinyliques ;
- les polymères éventuellement modifiés d'origine naturelle, tels que :
    - la gomme arabique, la gomme de guar, les dérivés du xanthane, la gomme de karaya ;
    - les alginates et les carraghénanes ;
    - les glycoaminoglycanes, l'acide hyaluronique et ses dérivés ;
    - la gomme laque, la gomme sandaraque, les dammars, l'élémis, les copals ;
    - l'acide désoxyribonucléique ;
    - les mucopolysaccharides, tels que l'acide hyaluronique, le sulfate de chondroïtine, et des mélanges de ceux-ci.

Ces polymères seront utilisés en particulier si l'on désire une élimination plus ou moins appréciable du film par de l'eau.

Afin d'améliorer la nature filmogène d'un polymère huileux ou aqueux, il est possible d'ajouter au système polymère un agent de coalescence qui sera choisi parmi les agents de coalescence connus.

**II. Filmogène siliconé**

***1) Polymère à squelette organique non siliconé greffé***

Ces polymères peuvent être liposolubles, lipodispersibles, hydrosolubles ou dispersibles en milieu aqueux, le cas échéant.

Les polymères ayant un squelette organique non siliconé greffé par des monomères contenant un polysiloxane sont constitués d'une chaîne organique principale formée de monomères organiques ne comprenant pas la silicone, sur laquelle on greffe, au sein de ladite chaîne ainsi qu'éventuellement sur au moins l'une des extrémités de celle-ci, au moins un macromère de polysiloxane.

Dans ce qui suit, on doit comprendre que l'expression «macromère de polysiloxane » désigne, ainsi qu'il est généralement accepté, tout monomère contenant une chaîne polymère du type polysiloxane dans sa structure.

Les monomères organiques non siliconés constituant la chaîne principale du polymère siliconé greffé peuvent être choisis parmi les monomères à insaturation éthylénique pouvant être polymérisés par la méthode radicalaire, les monomères polymérisables par polycondensation tels que ceux formant les polyamides, les polyesters, les polyuréthanes, les monomères à cycle ouvrant tels que ceux du type oxazoline ou caprolactone.

Les polymères ayant un squelette organique non siliconé greffé par des monomères contenant un polysiloxane selon la présente invention peuvent être obtenus conformément à toute méthode connue de l'homme de l'art, en particulier par la réaction entre (i) un macromère de polysiloxane de départ correctement fonctionnalisé sur la chaîne de polysiloxane et (ii) un ou plusieurs composés organiques non siliconés, eux même correctement fonctionnalisés par une fonction qui est capable de réagir avec le groupement ou les groupements fonctionnel(s) porté(s) par ladite silicone en formant une liaison covalente ; un exemple classique d'une telle réaction est la réaction radicalaire entre un groupement vinyle porté à l'une des extrémités de la silicone avec une double liaison d'un monomère à insaturation éthylénique de la chaîne principale.

Les polymères ayant un squelette organique non siliconé greffé par des monomères contenant un polysiloxane selon l'invention sont choisis de préférence parmi ceux décrits dans les brevets US 4 693 935, US 4 728 571 et US 4 972 037 et les demandes de brevet EP-A-0 412 704, EP-A-0 412 707, EP-A-0 640 105 et WO 95/00578. Il concerne des copolymères obtenus par la polymérisation radicalaire à partir de monomères à insaturation éthylénique et de monomères ayant un groupement terminal vinyle, ou bien des copolymères obtenus par la réaction d'une polyoléfine contenant des groupements fonctionnalisés et un macromère de polysiloxane ayant une fonction terminale réactive avec lesdits groupements fonctionnalisés.

Une famille particulière de polymères siliconés greffés convenables pour la mise en oeuvre de la présente

invention est constituée par les polymères siliconés greffés contenant :

a) de 0 à 98% en poids d'au moins un monomère lipophile (A) de faible polarité lipophile à insaturation éthylénique, polymérisable par la méthode radicalaire ;
b) de 0 à 98% en poids d'au moins un monomère polaire hydrophile (B) à insaturation éthylénique, copolymérisable avec le monomère ou les monomères du type (A) ;
c) de 0,01 à 50% en poids d'au moins un macromère de polysiloxane (C) de formule générale (XXVII) :

$$X(Y)_nSi(R)_{3-m}Z_m \qquad (XXVII)$$

dans laquelle :

- X désigne un groupement vinyle copolymérisable avec les monomères (A) et (B) ;
- Y désigne un groupement ayant une liaison divalente ;
- R désigne hydrogène, alkyle ou alkoxy en $C_1$-$C_6$, aryle en $C_6$-$C_{12}$ ;
- Z désigne un motif polysiloxane monovalent ayant un poids moléculaire moyen en nombre d'au moins 500 ;
- n vaut 0 ou 1 et m est un nombre entier allant de 1 à 3 ; les pourcentages étant calculés par rapport au poids total des monomères (A), (B) et (C).

Ces polymères ont un poids moléculaire moyen en nombre allant de 10 000 à 2 000 000, et préférablement une température de transition vitreuse Tg ou une température de fusion cristalline Tm d'au moins -20°C.
Comme exemples de monomères lipophiles (A), on peut citer les esters d'alcool en $C_1$-$C_{18}$ et de l'acide acrylique ou méthacrylique ; les esters d'alcool en $C_{12}$-$C_{30}$ et de l'acide méthacrylique, le styrène ; les macromères de polystyrène ; l'acétate de vinyle ; le propionate de vinyle ; l'alpha-méthylstyrène ; le tertio-butylstyrène ; le butadiène ; le cyclohexadiène; l'éthylène ; le propylène ; le vinyltoluène, les esters de l'acide acrylique ou méthacrylique et de 1,1-dihydroperfluoroalcanols ou d'homologues de ceux-ci ; les esters de l'acide acrylique ou méthacrylique et d'omega-hydrofluoroalcanols ; les esters de l'acide acrylique ou méthacrylique et de fluoroalkylsulfonamidoalcools ; les esters de l'acide acrylique ou méthacrylique et de fluoroalkylalcools ; les esters de l'acide acrylique ou méthacrylique et d'alcoolfluoroéthers ; ou des mélanges de ceux-ci. Les monomères (A) préférés sont choisis au sein du groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, l'acrylate de 2-(N-méthylperfluorooctanesulfonamido)éthyle, l'acrylate de 2-(N-butylperfluorooctanesulfonamido)éthyle, ou des mélanges de ceux-ci.
Comme exemples de monomères (B) polaires, on peut citer l'acide acrylique, l'acide méthacrylique, le N,N-diméthylacrylamide, le méthacrylate de diméthylamino-éthyle, le méthacrylate de diméthylaminoéthyle quaternisé, le (méth)acrylamide, le N-t-butylacrylamide, l'acide maléique, l'anhydride maléique et les hemi-esters de ceux-ci, les (méth)acrylates d'hydroxyalkyle, le chlorure de diallyldiméthylammonium, la vinyl-pyrrolidone, les éthers vinyliques, les maléimides, la vinylpyridine, le vinylimidazole, les composés polaires vinyliques et hétérocycliques, le sulfonate de styrène, l'alcool allylique, l'alcool vinylique, le vinylcaprolactame ou des mélanges de ceux-ci. Les monomères (B) sont choisis de préférence au sein du groupe constitué par l'acide acrylique, le N,N-diméthylacrylamide, le méthacrylate de diméthylaminoéthyle, le méthacrylate de diméthylaminoéthyle quaternisé, la vinylpyrrolidone, et des mélanges de ceux-ci.
On cite notamment le produit KP 561 ou le KP 562 commercialisé par SHIN ETSU tel que le monomère (A) et choisi parmi les esters d'alcool en $C_{18}$-$C_{22}$ et de l'acide méthacrylique.
Les macromères de polysiloxane (C) de formule (XXVII) sont choisis de préférence parmi ceux correspondant à la formule générale (XXVIII) suivante :

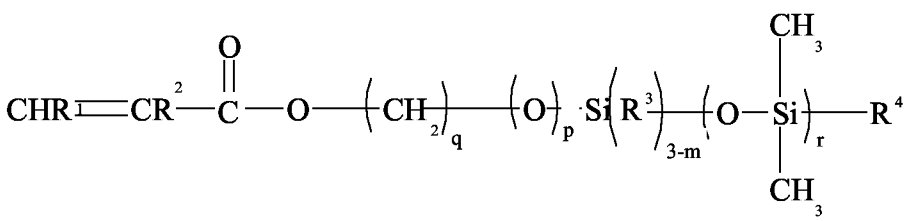
(XXVIII)

dans laquelle :

- $R^1$ est hydrogène ou -COOH (préférablement hydrogène) ;
- $R^2$ est hydrogène, méthyle ou $-CH_2COOH$ (préférablement méthyle) ;

- $R^3$ est alkyle, alkoxy ou alkylamino en $C_1$-$C_6$, aryle en $C_6$-$C_{12}$ ou hydroxyle (préférablement méthyle) ;
- $R^4$ est alkyle, alkoxy ou alkylamino en $C_1$-$C_6$, aryle en $C_6$-$C_{12}$ ou hydroxyle (préférablement méthyle) ;
- q est un nombre entier allant de 2 à 6 (préférablement 3) ;
- p vaut 0 ou 1 ;
- r est un nombre entier allant de 5 à 700 ;
- m est un nombre entier allant de 1 à 3 (préférablement 1).

On utilise de préférence les macromères de polysiloxane de formule (XXIX) :

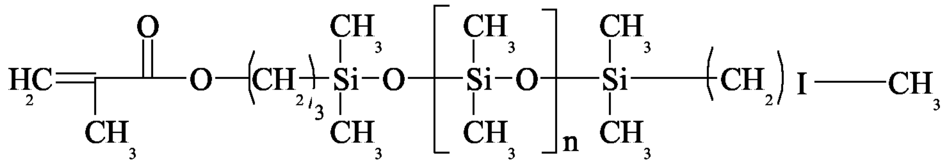 (XXIX)

n étant un nombre allant de 5 à 700 et I étant un nombre entier compris entre 0 et 3.

Un mode de réalisation de l'invention consiste en l'utilisation d'un copolymère capable d'être obtenu par une polymérisation radicalaire à partir du mélange de monomères constitué par :

a) 60% en poids d'acrylate de tertio-butyle ;
b) 20% en poids d'acide acrylique ;
c) 20% en poids de macromère siliconé de formule (XXX) :

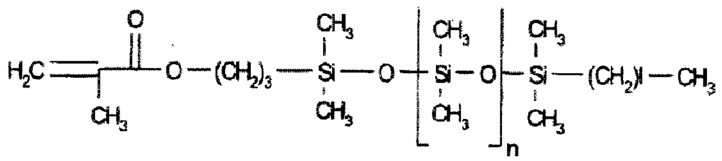 (XXX)

n étant un nombre allant de 5 à 700 et I étant un nombre entier compris entre 0 et 3, les pourcentages en poids étant calculés par rapport au poids total des monomères.

Un autre mode de réalisation particulier de l'invention consiste en l'utilisation d'un copolymère capable d'être obtenu par une polymérisation radicalaire à partir du mélange de monomères constitué par :

a) 80% en poids d'acrylate de tertio-butyle ;
b) 20% en poids de macromère siliconé de formule (XXXI) :

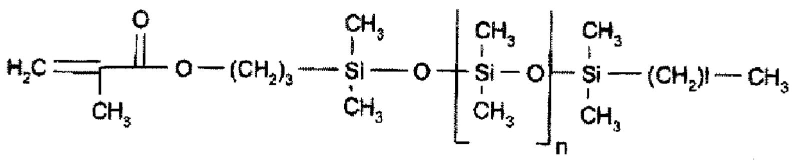 (XXXI)

n étant un nombre allant de 5 à 700 et I étant un nombre entier compris entre 0 et 3, les pourcentages en poids étant calculés par rapport au poids total des monomères.

Une autre famille particulière de polymères siliconés greffés ayant un squelette organique non siliconé convenable pour une mise en oeuvre de la présente invention est constituée par les copolymères siliconés greffés capables d'être obtenus par l'extrusion réactive d'un macromère de polysiloxane à fonction terminale réactive sur un polymère du type polyoléfine comprenant des groupements réactifs capables de réagir avec la fonction terminale du macromère de polysiloxane pour former une liaison covalente permettant le greffage de la silicone sur la chaîne principale de la polyoléfine. Ces polymères, ainsi que leur procédé de préparation, sont décrits dans la demande de brevet WO 95/00578.

Les polyoléfines réactives sont choisies de préférence parmi les polyéthylènes ou les polymères de monomères dérivés de l'éthylène, tels que le propylène, le styrène, l'alkylstyrène, le butylène, le butadiène, les (méth)acrylates, les esters vinyliques ou équivalents, comprenant des fonctions réactives capables de réagir avec la fonction terminale du macromère de polysiloxane. Ils sont choisis plus particulièrement parmi les copolymères d'éthylène ou de dérivés

d'éthylène et les monomères choisis parmi ceux comprenant une fonction carboxylique tels que l'acide (méth)acrylique ; ceux comprenant une fonction anhydride d'acide tels que l'anhydride de l'acide maléique ; ceux comprenant une fonction chlorure d'acide tels que le chlorure de l'acide (méth)acrylique ; ceux comprenant une fonction ester tels que les esters de l'acide (méth)acrylique ; et ceux comportant une fonction isocyanate.

Les macromères siliconés sont choisis de préférence parmi les polysiloxanes comprenant un groupement fonctionnalisé, à l'extrémité de la chaîne de polysiloxane ou à proximité de l'extrémité de ladite chaîne, choisis au sein du groupe constitué par les alcools, les thiols, les époxy, les amines primaires et secondaires, et tout particulièrement parmi ceux correspondant à la formule générale (XXXII):

$$T\text{-}(CH_2)_6\text{-}Si\text{-}[\text{-}(OSiR^5R^6)_t\text{-}R^7]_y \qquad (XXXII)$$

dans laquelle T est choisi parmi le groupe constitué par $NH_2$, NHRN, une fonction époxy, OH, SH ; $R^5$, $R^6$, $R^7$ et RN, indépendamment, désignent alkyle en $C_1$-$C_6$, phényle, benzyle ou alkylphényle en $C_6$-$C_{12}$, hydrogène ; s est un nombre allant de 2 à 00, t est un nombre allant de 0 à 1000 et y est un nombre allant de 1 à 3. Ils ont un poids moléculaire moyen en nombre allant préférablement de 5000 à 300 000, plus préférablement de 8000 à 200 000, et plus particulièrement de 9000 à 40 000.

Selon un mode de réalisation particulier, le polymère filmogène peut être obtenu auprès de la MINNESOTA MINING AND MANUFACTURING COMPANY sous les dénominations commerciales de polymères « SILICONE PLUS ». Par exemple, le poly(méthacrylate d'isobutyle-co-FOSEA de méthyle)-g-poly(diméthylsiloxane) est commercialisé sous la dénomination commerciale SA 70-5 IBMMF.

***2) Polymère à squelette siliconé***

Ledit polymère ou lesdits polymères siliconé(s) greffé(s) ayant un squelette de polysiloxane greffé par des monomères organiques non siliconés contenant une chaîne principale de silicone (ou de polysiloxane ($/SiO\text{-})_n$) sur laquelle se trouve greffé, à l'intérieur de ladite chaîne ainsi qu'éventuellement à l'une au moins de ses extrémités, au moins un groupement organique ne comprenant pas de silicone.

Les polymères ayant un squelette de polysiloxane greffé par des monomères organiques non siliconés selon l'invention peuvent être des produits commerciaux existants ou bien ils peuvent être obtenus par tout moyen connu de l'homme de l'art, en particulier par une réaction entre (i) une silicone de départ correctement fonctionnalisée sur un ou plusieurs de ces atomes de silicium et (ii) un composé organique non siliconé lui-même correctement fonctionnalisé par une fonction qui est capable de réagir avec le groupement ou les groupements fonctionnel(s) porté(s) par ladite silicone en formant une liaison covalente ; un exemple classique d'une telle réaction est la réaction d'hydrosylilation entre des groupements /Si-H et des groupements vinyliques $CH_2$=CH-, voire la réaction entre des groupements thio-fonctionnels -SH avec ces mêmes groupements vinyle.

Des exemples de polymères ayant un squelette de polysiloxane greffé par des monomères organiques non siliconés convenables pour une mise en oeuvre de la présente invention, ainsi que leur méthode spécifique de préparation, sont décrits en particulier dans les demandes de brevet EP-A-0 582 152, WO 93/23009 et WO 95/03776, dont les enseignements sont totalement inclus dans la présente description à titre de références non limitatives.

Selon un mode de réalisation particulièrement préféré de la présente invention, le polymère siliconé, ayant un squelette de polysiloxane greffé par des monomères organiques non siliconés, mis en oeuvre, est constitué du résultat d'une copolymérisation radicalaire entre, d'une part, au moins un monomère organique anionique non siliconé à insaturation éthylénique et/ou un monomère organique hydrophobe non siliconé à insaturation éthylénique et, d'autre part, une silicone présentant dans sa chaîne au moins un groupement fonctionnel, et préférablement plusieurs, capable de réagir avec lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou comme mélanges, parmi les acides carboxyliques insaturés, linéaires ou ramifiés, éventuellement neutralisés partiellement ou totalement sous forme d'un sel, ce ou ces acide(s) carboxylique(s) insaturé(s) pouvant être plus particulièrement l'acide acrylique, l'acide méthacrylique, l'acide maléique, l'acide itaconique, l'acide fumarique et l'acide crotonique. Les sels convenables sont en particulier les sels alcalins, alcalino-terreux et d'ammonium. On notera que, de même, dans le polymère siliconé greffé final, le groupement organique de nature anionique qui est constitué du résultat de l'(homo)polymérisation radicalaire d'au moins un monomère anionique du type acide carboxylique insaturé peut être, après réaction, post-neutralisé par une base (soude, ammoniaque, etc.) pour l'amener sous la forme d'un sel.

Selon la présente invention, les monomères hydrophobes à insaturation éthylénique sont de préférence choisis, seuls ou comme mélange, parmi les esters de l'acide acrylique d'alcanols et/ou les esters de l'acide méthacrylique d'alcanols. Les alcanols sont de préférence en $C_1$ à $C_{30}$ et plus particulièrement en $C_1$ à $C_{22}$. Les monomères préférés sont choisis parmi le groupe constitué par le (méth)acrylate d'isooctyle, le (méth)acrylate d'isononyle, le (méth)acrylate

de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)-acrylate de tridécyle et le (méth)acrylate de stéaryle, ou des mélanges de ceux-ci.

Une famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (XXXIII) ci-dessous :

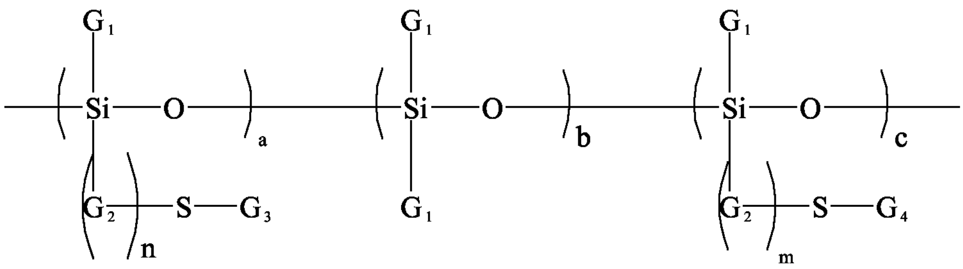

(XXXIII)

dans laquelle les radicaux $G_1$, identiques ou différents, représentent hydrogène ou un radical alkyle en $C_1$-$C_{10}$, voire un radical phényle ; les radicaux $G_2$, identiques ou différents, représentent un groupement alkylène en $C_1$-$C_{10}$ ; $G_3$ représente un reste polymère résultant de l'(homo)polymérisation d'au moins un monomère anionique à insaturation éthylénique ; $G_4$ représente un reste polymère résultant de l'(homo)polymérisation d'au moins un monomère d'au moins un monomère hydrophobe [sic] à insaturation éthylénique ; m et n sont égaux à 0 ou 1 ; a est un nombre entier allant de 0 à 50 ; b est un nombre entier pouvant être compris entre 10 et 350, c est un nombre entier allant de 0 à 50 ; à condition que l'un des paramètres a et c soit différent de 0.

Le motif de formule (XXXIII) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène ;
- $G_3$ représente un radical polymère résultant de l'(homo)polymérisation d'au moins un monomère du type acide carboxylique à insaturation éthylénique, préférablement l'acide acrylique et/ou l'acide méthacrylique ;
- $G_4$ représente un radical polymère résultant de l'(homo)polymérisation d'au moins un monomère du type (méth)acrylate d'alkyle en $C_1$-$C_{10}$, préférablement le (méth)acrylate d'isobutyle ou de méthyle.

Des exemples de polymères siliconés correspondant à la formule (XXXIII) sont en particulier des polydiméthylsiloxanes (PDMS) sur lesquels sont greffés, par l'intermédiaire d'une liaison secondaire du type thiopropylène, des motifs polymères mixtes du type acide poly(méth)acrylique et du type poly(méth)acrylate d'alkyle.

D'autres exemples de polymères siliconés correspondant à la formule (XXXIII) sont en particulier des polydiméthylsiloxanes (PDMS) sur lesquels sont greffés, par l'intermédiaire d'une liaison secondaire du type thiopropylène, des motifs polymères du type poly(méth)acrylate d'isobutyle.

De tels polymères comportent les polymères comprenant au moins un groupement de formule (XXXIV) :

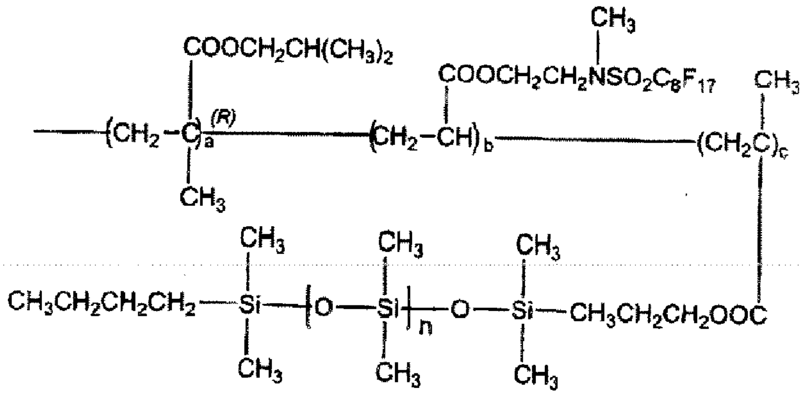

(XXXIV)

dans laquelle :

a, b et c, pouvant être identiques ou différents, sont chacun un nombre allant de 1 à 100 000 ; et les groupements terminaux, pouvant être identiques ou différents, sont choisis chacun parmi les groupements alkyle linéaires en $C_1$ à $C_{20}$, les groupements alkyle à chaîne ramifiée en $C_3$ à $C_{20}$, les groupements aryle en $C_3$ à $C_{20}$, les groupements alkoxy linéaires en $C_1$ à $C_{20}$ et les groupements alkoxy ramifiés en $C_3$ à $C_{20}$.

De tels polymères sont divulgués dans les brevets US n° 4 972 037, 5 061 481, 5 209 924, 5 849 275 et 6 033 650, et WO 93/23446 et WO 95/06078.

Une autre famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (XXXV) ci-dessous :

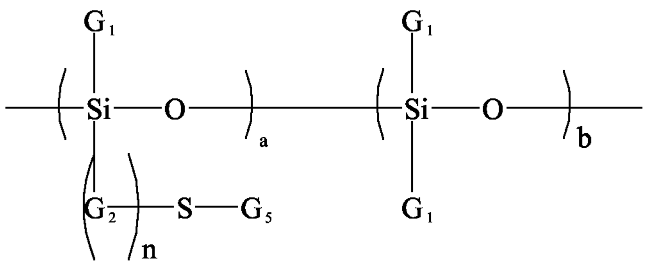

(XXXV)

dans laquelle les radicaux $G_1$ et $G_2$ ont la même signification que ci-dessus ; $G_5$ représente un reste polymère résultant de l'(homo)polymérisation d'au moins un monomère hydrophobe à insaturation éthylénique ou de la copolymérisation d'au moins un monomère anionique à insaturation éthylénique et d'au moins un monomère hydrophobe à insaturation éthylénique ; n est égal à 0 ou 1 ; a est un nombre entier allant de 0 à 50 ; b est un nombre entier pouvant être compris entre 10 et 350 ; à condition que a soit différent de 0.

Le motif de formule (XXXV) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène.

La masse moléculaire en nombre des polymères siliconés ayant un squelette de polysiloxane greffé par des monomères organiques non siliconés de l'invention varie préférablement d'environ 10 000 à 1 000 000, et encore plus préférablement d'environ 10 000 à 100 000.

Selon un mode de réalisation particulier, un polymère filmogène siliconé convenant particulièrement à la mise en oeuvre de la présente invention, peut être un copolymère comportant des groupements carboxylates et des groupements polydiméthylsiloxanes.

Par «copolymère comportant des groupements carboxylates et des groupements polydiméthylsiloxanes », on entend dans la présente demande, un copolymère obtenu à partir de (a) un ou plusieurs monomères carboxyliques (acide ou ester), et (b) une ou plusieurs chaînes polydiméthylsiloxane (PDMS).

On entend dans la présente demande par « monomère carboxylique » aussi bien les monomères d'acide carboxylique que les monomères d'ester d'acide carboxylique. Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : acrylate, méthacrylate, maléate, fumarate, itaconoate et/ou crotonoate. Les monomères sous forme d'esters sont plus particulièrement choisis parmi les acrylates et méthacrylates d'alkyle linéaire ou ramifié de préférence en $C_1$-$C_{24}$ et mieux en $C_1$-$C_{22}$, le radical alkyle étant préférentiellement choisi parmi les radicaux méthyle, éthyle, stéaryle, butyle, éthyl-2-hexyle, et leurs mélanges.

Le copolymère peut comprendre comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

On entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium. Les chaînes PDMS pouvant être utilisées

pour obtenir le copolymère comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe $CH_2 = CR_1 - CO - O - R_2$, où $R_1$ représente un hydrogène ou un groupe méthyle, et $R_2$ représente $-CH_2-$, $-(CH_2)_n-$ avec n = 3, 5, 8 ou 10, $-CH_2-CH(CH_3)-CH_2-$, $-CH_2-CH_2-O-CH_2-CH_2-$, $-CH_2-CH_2-O-CH_2-CH_2-CH(CH_3)-CH_2-$, $-CH_2-CH_2-O-CH_2$ $CH_2-O-CH_2-CH_2-CH_2-$.

Les copolymères utilisés sont généralement obtenus selon les méthodes usuelles de polymérisation et de greffage, par exemple par polymérisation radicalaire (A) d'un PDMS comportant au moins un groupe radical polymérisable (par exemple sur l'une des extrémités de la chaîne ou sur les deux) et (B) d'au moins un monomère carboxylique, comme décrit par exemple dans les documents US-A-5,061,481 et US-A-5,219,560.

Les copolymères obtenus ont généralement un poids molécule allant d'environ 3000 à 200 000 et de préférence d'environ 5000 à 100 000.

Le copolymère peut se présenter tel quel ou sous forme dispersée dans un solvant tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier comme copolymère utilisable, les copolymères commercialisés par la société SHIN-ETSU sous les dénominations KP-561 (nom CTFA : acrylates/dimethicone), KP-541 où le copolymère est dispersé à 60 % en poids dans de l'alcool isopropylique (nom CTFA : acrylates/dimethicone and Isopropyl alcohol), KP-545 où le copolymère est dispersé à 30 % dans du cyclopentasiloxane (nom CTFA : acrylates/dimethicone and Cyclopentasiloxane). Selon un mode préféré de réalisation de l'invention, on utilise de préférence le KP561 ; ce copolymère n'est pas dispersé dans un solvant, mais se présente sous forme cireuse, son point de fusion étant d'environ 30 °C.

Plus généralement, la quantité totale de polymère doit être en quantité suffisante pour former sur la peau et/ou les lèvres un film cohésif capable de suivre les mouvements de la peau et/ou des lèvres sans se décoller ou craquer.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

**<u>Particules ayant une susceptibilité magnétigue non nulle (particules magnétiques)</u>**

Par « particules magnétiques », encore appelées corps magnétiques, on désigne des particules présentant une susceptibilité magnétique, c'est-à-dire sensible à l'action d'un champ magnétique et tendant par exemple à s'aligner sur les lignes de champ.

La composition peut comporter à la fois des particules magnétiques et des particules non magnétiques.

La présence de particules magnétiques et de particules non magnétiques dans la composition peut permettre par exemple de créer de nouveaux effets optiques, modulables sous l'effet d'un champ magnétique.

De préférence, les particules magnétiques utilisées ne présentent pas d'aimantation rémanente en l'absence de champ magnétique.

Les particules magnétiques peuvent comporter tout matériau magnétique présentant une sensibilité aux lignes d'un champ magnétique, qu'il soit produit par un aimant permanent ou issu d'une induction, ce matériau étant par exemple choisi parmi le nickel, le cobalt, le fer, leurs alliages et oxydes, notamment $Fe_3O_4$, et aussi le gadolinium, le terbium, le dysprosium, l'erbium, leurs alliages et oxydes. Le matériau magnétique peut être de type « doux » ou « dur », et notamment comporter du fer métal, notamment du fer doux, éventuellement enrobé.

Les particules magnétiques peuvent présenter ou non une structure multicouche, comportant au moins une couche d'un matériau magnétique, tel que par exemple le fer, le nickel, le cobalt, leurs alliages et oxydes, notamment $Fe_3O_4$.

Les particules magnétiques sont de préférence asphériques, présentant par exemple une forme allongée. Ainsi, lorsque ces particules sont soumises au champ magnétique, elles tendent à s'orienter avec leur axe longitudinal dans l'alignement des lignes de champ, et subissent un changement d'orientation qui se traduit par un changement d'aspect de la première composition.

Lorsque les particules magnétiques sont sensiblement sphériques, de préférence leur aspect est inhomogène, de manière à ce qu'un changement d'orientation induise un changement d'aspect.

La quantité de particules magnétiques est suffisante pour que l'aspect de la composition puisse dépendre de

leur orientation et/ou de leur emplacement.

La concentration en particules magnétiques est par exemple comprise entre environ 0,05 et environ 97 % en masse, notamment entre environ 0,1 et environ 95 % en masse, mieux entre environ 0,1 et environ 90 % en masse, par exemple de l'ordre de 3 % en masse. La dimension des particules magnétiques est par exemple comprise entre 1 nm et 700 μm, mieux entre 1 μm et 500 μm, mieux encore entre 10 μm et 150 μm. Par « dimension », on désigne la dimension donnée par la distribution granulométrique statistique à la moitié de la population, dite D50.

**Pigments magnétiques**

Les particules magnétiques de la première composition peuvent comporter des pigments magnétiques. Des pigments convenant tout particulièrement sont les nacres comportant de l'oxyde de fer $Fe_3O_4$. Des pigments présentant des propriétés magnétiques sont par exemple ceux commercialisés sous les dénominations commerciales COLORONA BLACKSTAR BLUE, COLORONA BLACKSTAR GREEN, COLORONA BLACKSTAR GOLD, COLORONA BLACKSTAR RED, CLOISONNE NU ANTIQUE SUPER GREEN, MICRONA MATTE BLACK (17437), MICA BLACK (17260), COLORONA PATINA SILVER (17289) et COLORONA PATINA GOLD (117288) de la société MERCK ou bien encore FLAMENCO TWILIGHT RED, FLAMENCO TWILIGHT GREEN, FLAMENCO TWILIGHT GOLD, FLAMENCO TWILIGHT BLUE, TIMICA NU ANTIQUE SILVER 110 AB, TIMICA NU ANTIQUE GOLD 212 GB, TIMICA NU-ANTIQUE COPPER 340 AB, TIMICA NU ANTIQUE BRONZE 240 AB, CLOISONNE NU ANTIQUE GREEN 828 CB, CLOISONNE NU ANTIQUE BLUE 626 CB, GEMTONE MOONSTONE G 004, CLOISONNE NU ANTIQUE RED 424 CB, CHROMALITE BLACK (4498), CLOISONNE NU ANTIQUE ROUGE FLAMBE (code 440 XB), CLOISONNE NU ANTIQUE BRONZE (240 XB), CLOISONNE NU ANTIQUE GOLD (222 CB) et CLOISONNE NU ANTIQUE COPPER (340 XB) de la société ENGELHARD.

On peut encore citer les particules d'oxyde de fer noir, par exemple celles commercialisées sous la dénomination SICOVIT noir E172 par la société BASF ou les particules à base de fer doux proposées sous la dénomination STAPA® WM IRON VP 041040 par la société ECKART.

Les particules magnétiques peuvent être des fibres, selon le résultat recherché.

Le terme « fibres » désigne des corps généralement allongés, présentant par exemple un facteur de forme allant de 3,5 à 2 500 ou de 5 à 500, par exemple de 5 à 150. Le facteur de forme est défini par le rapport L/D, où L est la longueur de la fibre et D le diamètre du cercle dans lequel s'inscrit la plus grande section transversale de la fibre.

La section transversale des fibres peut s'inscrire par exemple dans un cercle de diamètre allant de 2 nm à 500 μm, par exemple allant de 100 nm à 100 μm, voire de 1 μm à 50 μm.

Les fibres peuvent présenter par exemple une longueur allant de 1 μm à 10 mm, par exemple de 0,1 mm à 5 mm, voire de 0,3 mm à 3,5 mm.

Les fibres peuvent présenter une masse allant par exemple de 0,15 à 30 deniers (masse en gramme pour 9 km de fil), par exemple de 0,18 à 18 deniers.

La forme en section transversale des fibres peut être quelconque, par exemple circulaire ou polygonale, notamment carrée, hexagonale ou octogonale.

La composition peut comporter des fibres pleines ou creuses, indépendantes ou liées entre elles, par exemple tressées.

La composition peut comporter des fibres ayant des extrémités épointées et/ou arrondies, par exemple par polissage.

Les fibres peuvent ne pas voir leur forme sensiblement modifiée lorsqu'elles sont introduites dans la composition, étant par exemple initialement rectilignes et suffisamment rigides pour conserver leur forme. En variante, les fibres peuvent présenter une souplesse leur permettant de se déformer sensiblement au sein de la composition.

Les fibres peuvent comporter une teneur non nulle, pouvant aller jusqu'à 100 %, d'un matériau magnétique choisi parmi les matériaux magnétiques doux, les matériaux magnétiques durs, notamment à base de fer, de zinc, de nickel, de cobalt ou de manganèse et leurs alliages et oxydes, notamment $Fe_3O_4$, les terres rares, le sulfate de baryum, les alliages de fer silicium, éventuellement chargés en molybdène, $Cu_2MnAl$, MnBi, ou un mélange de ceux-ci, cette liste n'étant pas limitative.

Lorsque la composition comporte des fibres contenant des particules magnétiques, ces dernières peuvent être présentes par exemple au moins à la surface de la fibre, voire à la surface des fibres uniquement, à l'intérieur de la fibre uniquement ou encore être dispersées au sein de la fibre de manière sensiblement homogène.

Les fibres peuvent comporter par exemple un coeur non magnétique avec une pluralité de particules magnétiques à sa surface.

Les fibres peuvent encore comporter une matrice synthétique contenant une pluralité de grains magnétiques dispersés en son sein.

Le cas échéant, une matière synthétique chargée de particules magnétiques peut elle-même être enrobée par une écorce non magnétique. Une telle écorce constitue par exemple une barrière isolant le ou les matériaux magnétiques

du milieu ambiant et/ou peut amener de la couleur. Les fibres peuvent comporter un coeur magnétique monolithique et être enrobées par une écorce non magnétique, ou cela peut être l'inverse.

La composition peut comporter des fibres réalisées par extrusion ou co-extrusion d'une ou plusieurs matières polymériques, notamment thermoplastiques et/ou élastomères. L'une des matières extrudées peut contenir une charge de particules magnétiques dispersées.

La fibre peut comporter une matière synthétique choisie parmi les polyamides, PET, acétates, polyoléfines, notamment PE ou PP, PVC, polyester bloc amide, Rilsan® plastifié, élastomères, notamment élastomères de polyester, élastomères de PE, élastomères de silicone, élastomères de nitrile ou un mélange de ces matériaux, cette liste n'étant pas limitative.

La composition peut contenir des fibres composites comportant un coeur magnétique enrobé au moins partiellement par au moins un matériau amagnétique, synthétique ou naturel. L'enrobage du coeur magnétique peut se faire par exemple par co-extrusion, autour du coeur, d'une écorce en un matériau non magnétique.

L'enrobage du coeur peut encore s'effectuer autrement, par exemple par polymérisation *in situ.*

Le coeur peut être monolithique ou comporter une charge de grains magnétiques dispersés dans une matrice.

La composition peut encore contenir des fibres composites obtenues par enrobage par une matière synthétique, chargée de particules magnétiques, d'un coeur amagnétique, synthétique ou naturel, le coeur étant composé par exemple d'une fibre de bois, de rayonne, de polyamide, d'une matière végétale, de polyoléfine, notamment de polyéthylène, de Nylon®, de polyimide-amide, d'aramide, cette liste n'étant pas limitative.

La composition peut encore comporter des particules composites magnétiques, notamment un latex magnétique.

**Particules composites magnétigues**

Une particule composite magnétique est un matériau composite constitué d'une matrice organique ou minérale et de grains magnétiques. Les particules composites magnétiques peuvent ainsi comporter à leur surface et/ou en leur sein des grains d'un matériau magnétique. Les particules composites peuvent être constituées d'un coeur magnétique enrobé d'une matrice organique ou minérale, ou inversement.

Les particules composites magnétiques comportent par exemple l'un des matériaux magnétiques précités.

La dimension des particules composites magnétiques est par exemple comprise entre 1 nm et 1 mm, mieux entre 100 nm et 500 μm, mieux encore entre 500 nm et 100 μm. Par « dimension », on désigne la dimension donnée par la distribution granulométrique statistique à la moitié de la population, dite D50.

La thèse de C. GOUBAULT, 23 Mars 2004, incorporée ici par référence, rappelle au chapitre 1 l'état de l'art en matière de particules composites magnétiques, et dresse une liste de procédés de préparation pouvant être utilisés pour préparer des particules composites magnétiques, à savoir une synthèse séparée des grains magnétiques et de la matrice, une synthèse des grains magnétiques au contact de la matrice ou une synthèse de la matrice en présence des grains magnétiques.

La société KISKER commercialise des particules magnétiques composites à matrice minérale, composée de silice. Les sociétés DYNAL, SERADYN, ESTAPOR et ADEMTECH proposent des particules magnétiques composites à matrice organique, susceptibles également d'être utilisées dans l'invention.

Plus particulièrement, la société ESTAPOR commercialise sous la référence M1-070/60 des latex magnétiques constitués de grains de ferrite uniformément répartis dans une matrice polystyrène, ce latex comportant 65 % d'oxyde de fer, le diamètre moyen des particules de polystyrène étant de 890 nm et la teneur massique en matières sèches de 10%.

**Ferrofluide**

La composition peut comporter un ferrofluide, c'est-à-dire une suspension colloïdale stable de particules magnétiques, notamment de nanoparticules magnétiques.

Les particules, d'une taille par exemple de l'ordre de quelques dizaines de nanomètres, sont dispersées dans un solvant (eau, huile, solvant organique), soit à l'aide d'un tensioactif ou d'un agent dispersant, soit par des interactions électrostatiques.

Les ferrofluides sont par exemple préparés par broyage de ferrites ou autres particules magnétiques jusqu'à l'obtention de nanoparticules qui sont ensuite dispersées dans un fluide contenant un surfactant, lequel s'adsorbe sur les particules et les stabilise, ou par précipitation en milieu basique d'une solution d'ions métalliques.

Chaque particule du ferrofluide présente un moment magnétique déterminé par la taille de la particule et par la nature du matériau magnétique.

Sous l'action d'un champ magnétique, les moments magnétiques des particules tendent à s'aligner suivant les lignes de champ, avec apparition d'une aimantation non nulle dans le liquide. Si le champ est annulé, il n'y a pas d'hystérésis et l'aimantation s'annule.

Au-delà d'une valeur seuil de champ, on peut également provoquer des changements macroscopiques dans le liquide, par exemple l'apparition de pics ou une modification des propriétés rhéologiques.

La dénomination « ferrofluide » englobe également une émulsion de gouttelettes de ferrofluide dans un solvant. Chaque goutte contient alors des particules magnétiques colloïdales en suspension stable. Cela permet de disposer d'un ferrofluide dans tout type de solvant. La dimension des particules magnétiques en suspension dans le ferrofluide est par exemple comprise entre 1 nm et 10 μm, mieux entre 1 nm et 1 μm, mieux encore entre 1 nm et 100 nm. Par « dimension », on désigne la dimension donnée par la distribution granulométrique statistique à la moitié de la population, dite D50.

On peut citer notamment les ferrofluides commercialisés par la société LIQUIDS RESEARCH LTD sous les références :

- WHKS1S9 (A, B ou C), qui est un ferrofluide à base aqueuse comportant de la magnetite ($Fe_3O_4$), ayant des particules de 10 nm de diamètre.
- WHJS1 (A, B ou C), qui est un ferrofluide à base d'iso-paraffine et de particules de magnetite ($Fe_3O_4$) de 10 nm de diamètre.
- BKS25_dextran, qui est un ferrofluide à base aqueuse stabilisé par du dextran, comportant des particules de magnetite ($Fe_3O_4$) de 9 nm de diamètre.

## Chaînes de particules et/ou de fibres magnétiques

La composition peut encore comporter des chaînes de particules et/ou de fibres magnétiques.

La composition peut ainsi comporter des agglomérats de particules ou fibres dont la plus grande dimension, par exemple la longueur, est par exemple comprise entre 1 nm et 10 mm, par exemple entre 10 nm et 5 mm, ou entre 100 nm et 1 mm, ou encore entre 0,5 μm et 3,5 mm, par exemple entre 1 μm et 150 μm. La dimension désigne celle donnée par la distribution granulométrique statistique à la moitié de la population, dite D50.

Des chaînes de particules magnétiques peuvent être obtenues par exemple en assemblant des particules magnétiques colloïdales, comme cela est décrit dans les publications « Permanently linked monodisperse paramagnetic chains », E.M. Furst, C. Suzuki, M. Fermigier, A.P. Gast, Langmuir, 14, 7334-7336 (1998), « Suspensions de particules magnétiques », M. Fermigier, Y. Grasselli, Bulletin de la SFP (105) juillet 96, et « Flexible magnetic filaments as micromechanical sensors », C. Goubault, P. Jop, M. Fermigier, J. Baudry, E. Bertrand, J. Bibette, Phys. Rev. Lett., 91, 26, 260802-1 à 260802-4 (2003), dont les contenus sont incorporés par référence.

Il est notamment décrit dans ces articles comment procéder pour obtenir des chaînes de particules de latex magnétiques comportant une matrice de polystyrène contenant des grains d'oxyde de fer et fonctionnalisées en surface, liées entre elles de façon permanente suite à une réaction chimique, notamment des liaisons covalentes entre les surfaces des particules adjacentes ; il est également décrit un procédé d'obtention de chaînes de gouttelettes d'émulsion de ferrofluides, liées entre elles par interactions de nature physique. La longueur ainsi que le diamètre des chaînes permanentes ainsi obtenues peuvent être contrôlés. De telles chaînes magnétiques constituent des objets magnétiques anisotropes orientables et déplaçables sous l'effet d'un champ magnétique.

Les dimensions des chaînes magnétiques peuvent répondre aux mêmes conditions que les fibres magnétiques.

D'une manière générale, les composés selon l'invention contiennent avantageusement une phase grasse et/ou une phase aqueuse, notamment telles que définies précédemment.

En ce qui concerne la phase grasse, celle-ci peut contenir des huiles autres que les huiles volatiles précitées, notamment des huiles hydrocarbonées ou siliconées non volatiles en association, le cas échéant, avec des matières grasses solides telles que des cires et/ou des composés pâteux.

## AUTRES COMPOSES

La composition peut comporter des particules réfléchissantes, notamment à reflet métallique.

## Particules réfléchissantes

Ces particules peuvent être de toute forme, par exemple de plaquette ou globulaire, notamment sphérique, allongée ou non, avec le cas échéant un facteur de forme importent, et peuvent présenter ou non une susceptibilité magnétique non nulle, liée à la présence d'un matériau magnétique.

Lorsque les particules réfléchissantes sont magnétiques, de préférence elles présentent une forme aplatie de telle sorte qu'une modification de leur orientation au sein de la composition induise un changement d'aspect.

Les particules réfléchissantes, quelle que soit leur forme, peuvent présenter une structure multicouche ou non et, dans le cas d'une structure multicouche, comporter par exemple au moins une couche ayant de préférence une

épaisseur uniforme, notamment d'un matériau réfléchissant, avantageusement un composé métallique.

Lorsque les particules réfléchissantes ne présentent pas de structure multicouche, elles peuvent être composées par exemple d'au moins un composé métallique, par exemple un oxyde métallique, notamment un oxyde de titane ou de fer obtenu par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant, notamment au moins une couche d'au moins un composé métallique tel qu'un métal ou alliage. La couche du composé métallique est avantageusement une couche extérieure de la structure.

Le substrat peut être monomatière, multimatériau, organique et/ou inorganique.

Plus particulièrement, le substrat peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

A titre d'exemple de particules réfléchissantes comportant un substrat minéral enrobé d'une couche de métal, on peut citer également les particules comportant un substrat de borosilicate enrobé d'argent. Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes, quelle que soit leur forme, peuvent également être choisies parmi les particules à substrat synthétique enrobé au moins partiellement d'au moins une couche d'au moins un oxyde métallique, choisi par exemple parmi les oxydes de titane, notamment $TiO_2$, de fer notamment $Fe_2O_3$, d'étain, de chrome, le sulfate de baryum et les matériaux suivants : $MgF_2$, $CrF_3$, ZnS, ZnSe, $SiO_2$, $Al_2O_3$, MgO, $Y_2O_3$, $SeO_3$, SiO, $HfO_2$, $ZrO_2$, $CeO_2$, $Nb_2O_5$, $Ta_2O_5$, $MoS_2$ et leurs mélanges.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Comme autres exemples de particules réfléchissantes présentant en surface un composé métallique ou incluant au moins un composé métallique enrobé, on peut citer les particules proposées sous les dénominations METASHINE® ME 2040 PS, METASHINE® MC5090 PS ou METASHINE® MC280GP (2523) par la société NIPPON SHEET GLASS, SPHERICAL SILVER POWDER® DC 100, SILVER FLAKE® JV 6 ou GOLD POWDER® A1570 par la société ENGELHARD, STARLIGHT REFLECTIONS FXM® par la société ENERGY STRATEGY ASSOCIATES INC, BRIGHT SILVER® 1 E 0.008X0.008 par la société MEADOWBROOK INVENTIONS, ULTRAMIN® (ALUMINIUM POUDRE FINE LIVING), et COSMETIC METALLIC POWDER VISIONNAIRE BRIGHT SILVER SEA®, COSMETIC METALLIC POWDER VISIONAIRE NATURAL GOLD® (60314) ou COSMETIC METALLIC POWDER VISIONAIRE HONEY® (60316) par la société ECKART.

Les particules réfléchissantes peuvent réfléchir le spectre visible de manière sensiblement uniforme, comme c'est le cas par exemple de particules revêtues d'un métal tel que l'argent ou l'aluminium, ou non, ce qui peut alors conduire par exemple à un reflet métallique ayant un ton non neutre, jaune, rose, rouge, bronze, orangé, brun, or et/ou cuivré, selon la nature par exemple du composé métallique de surface.

Les particules réfléchissantes peuvent être présentes dans la composition à une teneur allant de 0,5 % à 60 % par rapport au poids total de la première composition, notamment de 1 % à 30 % en poids, par exemple de 3 % à 10 % en poids.

La composition peut comporter au moins un pigment diffractant.

## Pigment diffractant

Par « pigment diffractant », on désigne un pigment capable de produire une variation de couleur selon l'angle d'observation lorsqu'éclairé par de la lumière blanche, en raison de la présence d'une structure qui diffracte la lumière. Un tel pigment est encore parfois appelé pigment holographique.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinusoïdaux, en escalier.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non, de même linéature ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : $MgF_2$, $SiO_2$, $Al_2O_3$, $AlF_3$, $CeF_3$, $LaF_3$, $NdF_3$, $SmF_2$, $BaF_2$, $CaF_2$, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs matériaux, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : $MgF_2$, SiO, $SiO_2$, $Al_2O_3$, $TiO_2$, WO, AIN, BN, $B_4C$, WC, TiC, TiN, $N_4Si_3$, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de $SiO_2$, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : $MgF_2/Al/MgF_2$, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du $MgF_2$ peut être comprise entre 80 et 95 % du poids total du pigment.

D'autres pigments diffractants sont commercialisés sous les dénominations Metalure® Prismatic par la société ECKART®.

La quantité de pigment diffractant peut varier, en poids par rapport au poids total de la première composition, par exemple de 0,1 à 5 %.

La dimension du pigment diffractant peut être comprise par exemple entre 5 et 200 $\mu$m, mieux entre 5 et 100 $\mu$m, par exemple entre 5 et 30 $\mu$m.

L'épaisseur des particules de pigment diffractant peut être inférieure ou égale à 3 $\mu$m, mieux 2 $\mu$m, par exemple de l'ordre de 1 $\mu$m.

<u>**Autres agents de coloration**</u>

La composition peut comporter au moins un agent de coloration produisant de la lumière par absorption d'au moins une partie du spectre visible.

Un tel agent de coloration produisant une couleur par un phénomène d'absorption peut être constitué par un pigment magnétique ou non, organique ou inorganique ou hybride comportant à la fois de la matière organique et de la matière inorganique.

L'agent de coloration peut être un composé particulaire ou non.

Le cas échéant, les particules d'un même pigment magnétique constituent à la fois l'agent de coloration produisant la couleur par un phénomène d'absorption et les corps magnétiques.

Lorsque l'agent de coloration comporte un colorant, celui-ci peut être choisi parmi les colorants liposolubles et

hydrosolubles.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent par exemple représenter de 0,1 à 20 % du poids de la première ou de la deuxième composition, voire de 0,1 à 6 %, lorsque présents.

L'agent de coloration peut encore être une laque ou un pigment organique choisi parmi les matériaux ci-dessous et leurs mélanges :

- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

L'agent de coloration peut être une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

La composition peut comporter un pigment composite, comportant un noyau enrobé au moins partiellement par une écorce.

**Pigments composites**

Un pigment composite peut être composé notamment de particules comportant :

- un noyau inorganique,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite peut présenter par exemple une surface spécifique comprise entre 1 et 1000 $m^2/g$, notamment entre 10 et 600 $m^2/g$ environ, et en particulier entre 20 et 400 $m^2/g$ environ. La surface spécifique est la valeur mesurée par la méthode BET.

La proportion massique du noyau peut excéder 50 % par rapport au poids total du pigment composite, par exemple aller de 50 % à 70 %, par exemple de 60 % à 70 %.

Le pigment composite peut être différent d'un pigment interférentiel tel que décrit par exemple dans le brevet US 6 428 773. Un pigment interférentiel comporte par exemple plusieurs couches d'épaisseurs constantes de matériaux sélectionnés pour pouvoir produire des interférences optiques.

La saturation C* du pigment composite peut être supérieure ou égale à 30, mesurée selon le protocole ci-dessous.

**Protocole de mesure de la saturation du vigment composite :**

Les valeurs a* et b* dans l'espace CIE L*a*b* du pigment composite sont mesurées comme suit :

Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3700d, en mode spéculaire exclu, sous illuminant D65, ouverture moyenne. La saturation est donnée par $C^* = (a^{*2} + b^{*2})^{1/2}$.

**Noyau inorganique**

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique peut présenter une taille moyenne comprise entre environ 1 nm et environ 100 nm, voire entre environ 5 nm et environ 75 nm, par exemple entre environ 10 nm et environ 50 nm, notamment 20 ou 25 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau inorganique peut présenter un indice de réfraction supérieur ou égal à 2, voire supérieur ou égal à 2,1, par exemple supérieur ou égal à 2,2.

Le noyau inorganique peut être réalisé dans un matériau magnétique ou non choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment $TiO_2$, de fer, notamment $Fe_2O_3$, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 $m^2/g$ et environ 1000 $m^2/g$, mieux entre environ 10 $m^2/g$ et environ 600 $m^2/g$, par exemple entre environ 20 $m^2/g$ et environ 400 $m^2/g$.

Le noyau inorganique peut être coloré, le cas échéant.

**Matière colorante organique**

La matière colorante organique peut comporter par exemple au moins un pigment organique, par exemple au moins une laque organique.

La matière colorante organique peut être choisie par exemple parmi les composés particulaires insolubles dans le milieu physiologiquement acceptable de la composition.

La matière colorante organique peut comporter par exemple des pigments, par exemple des laques organiques ou autres matières colorantes organiques, qui peuvent être choisis parmi les composés ci-dessous et leurs mélanges :

- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par «The Cosmetic, Toiletry, and Fragrance Association », dont le contenu est incorporé dans la présente demande par référence.

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

La teneur totale en matière colorante organique de la composition, provenant du pigment composite et d'autres pigments éventuels, peut être par exemple inférieure à 10%, par rapport au poids total de la composition.

La proportion de la matière colorante organique peut excéder 30 % par rapport au poids total du pigment composite, par exemple aller de 30 à 50 %, par exemple de 30 à 40%.

## Liant

Le liant du pigment composite peut être de tout type dès lors qu'il permet à la matière colorante organique d'adhérer à la surface du noyau inorganique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :

- les organosilanes (1) obtenus à partir d'alkoxysilanes,
- les polysiloxanes (2) modifiés ou non choisis parmi une liste non limitative comprenant :
- les polysiloxanes modifiés (2A) comprenant au moins un radical choisi parmi, notamment, les polyéthers, les polyesters et les composés époxy (ils seront appelés « polysiloxanes modifiés »),

- les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

$$R^1_a\ Si\ X_{4-a} \qquad (I)$$

dans laquelle :

- $R^1$ représente $C_6H_5$-, $(CH_3)_2$ CH-$CH_2$- ou un radical de type $C_b$ $H_{2b+1}$- (où b varie de 1 à 18),
- X représente $CH_3O$- ou $C_2H_5O$-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) :

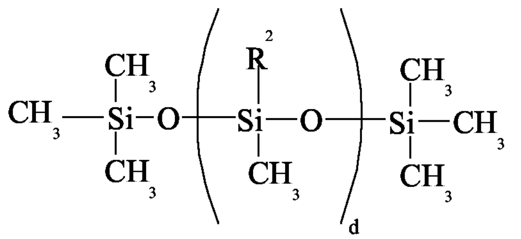

(II)

dans laquelle $R^2$ représente H- ou $CH_3$- et d varie de 15 à 450.

Parmi ces polysiloxanes, ceux pour lesquels $R^2$ représente H sont préférés.

Les polysiloxanes modifiés (2A) peuvent notamment répondre aux formules suivantes :

- ($a^1$) polysiloxanes modifiés portant des polyéthers, représentés par la formule (III)

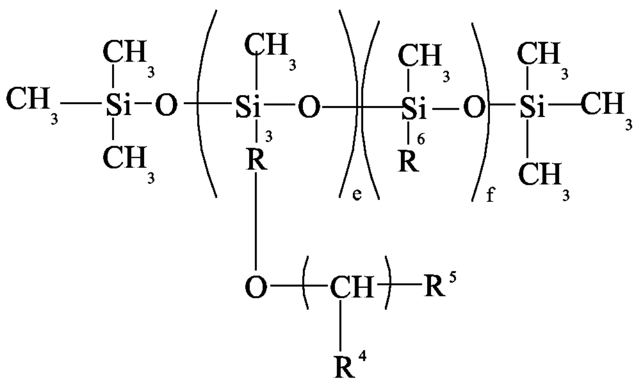

(III)

dans laquelle $R^3$ représente $-(CH_2)_h-$ ; $R^4$ représente $-(CH_2)_i-$ $CH_3$ ; $R^5$ représente -OH, - COOH, -CH = $CH_2$, -C ($CH_3$) = $CH_2$ ou $-(CH_2)_j-$ $CH_3$ ; $R^6$ représente $-(CH2)_k-$ $CH_3$ ; g et h variant indépendamment de 1 à 15 ; j et k variant indépendamment de 0 à 15 ; e variant de 1 à 50 et f variant de 1 à 300,

- ($a^2$) polysiloxanes modifiés portant des polyesters, représentés par la formule (IV) :

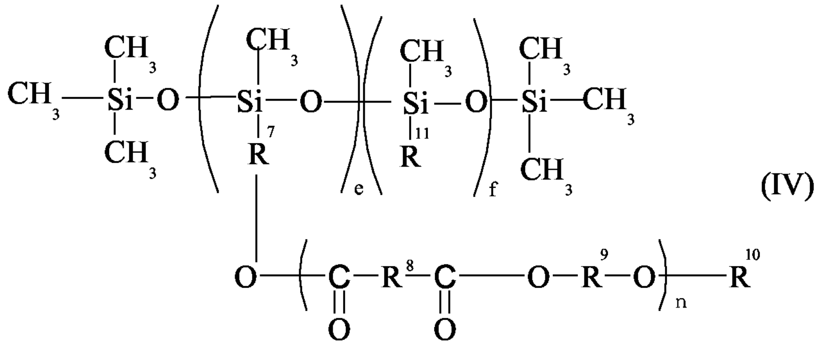

(IV)

dans laquelle $R^7$, $R^8$ et $R^9$ représentent indépendamment $-(CH_2)_q-$ ; $R^{10}$ représente -OH ; -COOH, -CH = $CH_2$, $-C(CH_3) = CH_2$ ou $-CH_2)_r- CH_3$ ; $R^{11}$ représente $-(CH_2)_s- CH_3$ ; n et q variant indépendamment de 1 à 15, r et s variant indépendamment de 0 à 15 ; e variant de 1 à 50 et f variant de 1 à 300,

- ($a^3$) polysiloxanes modifiés portant des radicaux époxy représentés par la formule (V):

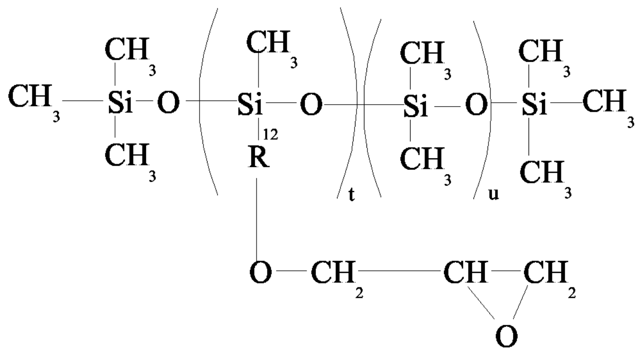

(V)

dans laquelle $R^{12}$ représente $-(CH_2)_v-$ ; v variant de 1 à 15 ; t variant de 1 à 50 et u variant de 1 à 300 ; ou leurs mélanges.

Parmi les polysiloxanes modifiés (2A), les polysiloxanes modifiés portant des polyéthers de formule (III) sont préférés.

Les polysiloxanes modifiés sur la partie terminale (2B) peuvent répondre à la formule (VI) :

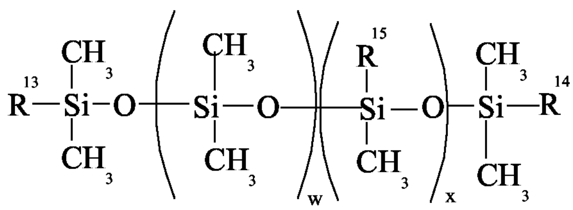

(VI)

dans laquelle $R^{13}$ et $R^{14}$ peuvent représenter -OH, $R^{16}$-OH ou $R^{17}$-COOH, indépendamment l'un de l'autre ; $R^{15}$ représente $-CH_3$ ou $-C_6H_5$ ; $R^{16}$ et $R^{17}$ représentent$-(CH_2)_y-$ ; y variant de 1 à 15 ; w variant de 1 à 200 et x variant de 0 à 100.

Parmi ces polysiloxanes modifiés sur au moins une extrémité, ceux portant au moins radical ($R^{16}$ et/ou $R^{17}$) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

$$CF_3(CF_2)_zCH_2CH_2 (R^{18})_a SiX_{4-a} \qquad (VII)$$

dans laquelle :

- $R^{18}$ représente $CH_3$-, $C_2H_5$-, $CH_3O$- ou $C_2H_5O$-,

- X représente $CH_3O$- ou $C_2H_5O$-,
- Z varie de 0 à 15 et a varie de 0 à 3.

Les fluoroalkylsilanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyltriéthoxysilane, le tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ-aminopropyl-triéthoxysilane, le γ-flycidoxypropyltriméthoxysilane, le γ-mercaptopropyltriméthoxysilane, le γ-méthacryloxypropyltriméthoxysilane, le N-β(aminoéthyl)-y-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthyl-aminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triéthylacétoacétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2. La proportion relative de liant peut être inférieure ou égale à 5 %, par exemple inférieure ou égale à 3 %, par rapport au poids total du pigment composite.

**<u>Préparation du pigment composite</u>**

Le pigment composite peut être préparé par tout procédé approprié, par exemple un procédé méchano-chimique ou un procédé de précipitation en solution, avec dissolution de la matière colorante organique puis précipitation à la surface du noyau.

Un liant peut être utilisé ou non.

Un procédé comportant un mélange mécanique d'un pigment organique et du noyau inorganique est préféré.

Un liant peut être ajouté et mélangé au noyau inorganique avant l'introduction de la matière colorante organique.

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Dans un exemple de mise en oeuvre, on commence par mélanger les particules destinées à constituer le noyau inorganique avec le liant.

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique

avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

Outre un agent de coloration absorbant la lumière par un phénomène d'absorption, la composition peut comporter au moins un pigment interférentiel ou diffractant, et/ou des particules réfléchissantes.

Dans un exemple de mise en oeuvre de l'invention, la première composition comporte au moins un agent de coloration goniochromatique qui permet d'observer un changement de couleur en fonction de l'angle d'observation. Cet agent de coloration goniochromatique peut être ou non magnétique.

Lorsque la première composition comporte des particules magnétiques d'une certaine couleur et un agent de coloration goniochromatique non magnétique, ce dernier peut être choisi de manière à ce que le trajet de couleur passe sensiblement par la couleur des particules magnétiques.

Cela peut permettre par exemple de rendre plus difficilement détectables les particules magnétiques à défaut d'une orientation de celles-ci sous l'effet d'un champ magnétique.

Cela peut permettre également de ne faire apparaître le motif induit par l'orientation des particules magnétiques que dans certaines conditions d'observation et/ou d'éclairage du support maquillé, ce qui peut permettre de créer des effets particulièrement attractifs d'apparition et de disposition du motif.

## Agents de coloration goniochromatiques

La composition peut contenir au moins un agent de coloration goniochromatique, lequel peut présenter des propriétés magnétiques, le cas échéant.

Par « agent de coloration goniochromatique », on désigne au sens de la présente invention un agent de coloration permettant d'obtenir, lorsque la composition est étalée sur un support, un trajet de couleur dans le plan a*b* de l'espace colorimétrique CIE 1976 qui correspond à une variation Dh de l'angle de teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la première composition a été étalée à l'état fluide avec une épaisseur de 300 $\mu$m au moyen d'un étaleur automatique sur une carte de contraste de marque ERICHSEN et de référence Typ 24/5, la mesure étant effectuée sur le fond noir de la carte.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques sont par exemple les structures suivantes : $Fe_2O_3/SiO_2/Fe_2O_3/SiO_2/Fe_2O_3$ un pigment ayant cette structure étant commercialisé sous la dénomination SICOPEARL par la société BASF ; $MoS_2/SiO_2$/mica-oxyde/$SiO_2/MoS_2$; $Fe_2O_3/SiO_2$/mica-oxyde/$SiO_2/Fe_2O_3$ ; $TiO_2/SiO_2/TiO_2$ et $TiO_2/Al_2O_3/TiO_2$, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE® HC par la société WACKER.

Comme agent de coloration goniochromatique, on peut encore utiliser certaines nacres, des pigments à effets sur substrat synthétique, notamment substrat type alumine, silice, borosilicate, oxyde de fer, aluminium, ou des paillettes holographiques interférentielles issues d'un film de polytéréphthalate.

## Nacres

Par « nacre », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites dans la composition, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La composition peut comporter par exemple au moins une charge, magnétique ou non.

## Charges

Par « charge », on désigne des particules de toute forme, insolubles dans le milieu de la composition, quelle que soit la température à laquelle la composition est fabriquée. Une charge peut servir notamment à modifier la rhéologie ou la texture de la composition. La nature et la quantité des particules pourra dépendre des propriétés mécaniques et des textures recherchées.

A titre d'exemple de charges, on peut citer, entre autres, le talc, le mica, la silice, le kaolin, la séricite, les poudres de polyamide, de polyoléfine, par exemple de polyéthylène, de polytétrafluoroéthylène, de polyméthacrylate de méthyle, de polyuréthane, les poudres d'amidon et les billes de résine de silicone.

## Actifs

La composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...), autobronzants. Ces actifs peuvent être utilisés par exemple à des concentrations de 0 à 20 % et notamment de 0,001 à 15 % par rapport au poids total de la composition.

La composition peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, les colorants ou leurs mélanges.

La composition selon l'invention peut comprendre, selon le type d'application envisagée, les constituants classiquement utilisés dans les domaines considérés, qui sont présents en une quantité appropriée à la forme galénique souhaitée.

## Formes galéniques

La composition peut se présenter sous diverses formes, en fonction de sa destination. La composition peut ainsi se présenter sous toute forme galénique normalement utilisée pour une application topique et notamment sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situées à l'interface huile/eau.

## EXEMPLES PROPOSES

Les proportions indiquées sont massiques sauf si le contraire est spécifié.

### Exemple A : Rouge à lèvres

| | |
|---|---|
| Polymère filmogène** | 65,24 |
| Sucrose acétate isobutyrate | 9,52 |
| Octyldodécanol | 6 |
| Isododécane | 14,1 |
| Pigment magnétiques* | 4,76 |
| Parfum | 0,38 |

* Pigment à base de fer doux STAPA® VM VP 041040 de la société ECKART.
** Polymère séquencé poly(acrylate d'isobornyle/méthacrylate d'isobornyle /acrylate d'isobutyle), obtenu comme suit

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 105 g d'acrylate d'isobornyle, 105 g de méthacrylate d'isobornyle, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'isobutyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobornyle) ayant une Tg de 110°C, une deuxième séquence polyacrylate d'isobutyle ayant une Tg de - 20°C et une séquence intermédiaire qui est un polymère statistique acrylate d'isobornyle/méthacrylate d'isobornyle/acrylate d'isobutyle.
Ce polymère présente une masse moyenne en poids de 100300 g/mol et une masse moyenne en nombre de 22800 g/mol, soit un indice de polydispersité I de 4.40. La composition de rouge à lèvres est préparée en chauffant les huiles non volatiles à 60 °C, sous agitation magnétique. Le polymère séquencé est introduire dans un bécher, ainsi que l'isododécane, et l'on place l'ensemble sous agitation rayneri. Quand on observe un mélange liquide transparent, on introduit le pigment magnétique et on laisse sous agitation rayneri pendant 20 minutes.
Une telle composition peut s'appliquer au moyen d'un applicateur tel qu'illustré à la figure 2.
Le séchage de la composition est suffisamment lent pour permettre la formation d'un motif par exposition au champ magnétique, lequel modifie l'orientation des particules de pigment magnétique. Néanmoins, la composition se fige assez rapidement pour que le motif n'ait pas le temps d'être détruit après suppression du champ.

### Exemple B : Vernis à ongles

| | |
|---|---|
| Nitrocellulose | 11 |
| N-éthyl o,p-toluènesulfonamide | 5 |
| Résine alkyde | 10 |

(suite)

| | |
|---|---|
| Isopropanol | 4 |
| Pigments magnétiques* | 0,5 |
| Acétate de butyle/acétate d'éthyle 50/50 | Qsp 100 |
| *Nacres contenant au moins 14 % de $Fe_3O_4$ de référence COLORONA PATINA GOLD (117288) commercialisées par la société MERCK. | |

L'aspect d'un tel vernis à ongles peut être modifié en appliquant un champ magnétique, avant que le vernis n'ait eu le temps de sécher.

L'expression « compris entre » doit se lire bornes incluses, sauf si le contraire est spécifié et «comportant un » doit être compris comme signifiant «comportant au moins un ».

## Revendications

**1.** Procédé de maquillage des matières kératiniques, notamment de la peau, des lèvres ou des phanères, comprenant les étapes suivantes :

(a) appliquer sur les matières kératiniques au moyen d'un applicateur cosmétique non magnétique une composition de maquillage comprenant

(i) au moins un solvant volatil,
(ii) des particules ayant une susceptible magnétique non nulle,

(b) soumettre le dépôt à un champ magnétique de manière à modifier l'orientation et/ou à déplacer au moins certaines desdites particules à susceptibilité magnétique non nulle,

le champ magnétique étant appliqué de manière à former au moins un motif sur la composition.

**2.** Procédé selon la revendication 1, dans lequel la composition comprend au moins un polymère filmogène.

**3.** Procédé selon la revendication 2, dans lequel le polymère filmogène est un polymère séquencé.

**4.** Procédé selon la revendication 3, dans lequel le polymère séquencé comporte au moins trois séquences distinctes.

**5.** Procédé selon l'une quelconque des revendications 1 à 4, dans lequel le solvant volatil est un solvant organique.

**6.** Procédé selon la revendication 5, dans lequel le solvant volatil comprend au moins une huile volatile.

**7.** Procédé selon la revendication 6, dans lequel l'huile volatile est une huile hydrocarbonée.

**8.** Procédé selon la revendication 6, dans lequel l'huile volatile est une huile siliconée.

**9.** Procédé selon la revendication 7, dans lequel l'huile volatile comprend de l'isododécane.

**10.** Procédé selon l'une quelconque des revendications 1 à 9, dans lequel les particules ayant une susceptibilité magnétique non nulle sont des pigments.

**11.** Procédé selon l'une quelconque des revendications précédentes, dans lequel les particules magnétiques comportent du fer métal, notamment du fer doux.

**12.** Procédé selon l'une quelconque des revendications précédentes, dans lequel le champ magnétique est exercé par un aimant permanent.

**13.** Procédé selon l'une quelconque des revendications 1 à 11, dans lequel le champ magnétique est exercé par un

électroaimant.

**14.** Procédé selon la revendication 12 ou 13, dans lequel le champ magnétique est rotatif.

**15.** Procédé selon l'une quelconque des revendications précédentes, dans lequel une région au moins du support revêtue de la composition est non exposée au champ magnétique.

**16.** Procédé selon l'une quelconque des revendications précédentes, dans lequel on laisse le dépôt sécher après l'application du champ magnétique.

**17.** Procédé selon la revendication 1, dans lequel le dépôt est formé sur les lèvres.

**18.** Procédé selon la revendication 1, dans lequel le dépôt est formé sur les ongles.

**19.** Kit de maquillage des matières kératiniques pour la mise en oeuvre d'un procédé selon la revendication 1, comportant :

- une composition de maquillage comprenant :

  (i) au moins un solvant volatil,
  (ii) des particules ayant une susceptible magnétique non nulle,

- un applicateur cosmétique non magnétique,
- un dispositif magnétique permettant de générer un champ magnétique,
- le dispositif magnétique étant apte à créer un champ magnétique susceptible, lorsque les matières kératiniques recouvertes d'un dépôt de ladite composition sont introduites dans ledit champ magnétique, de modifier l'orientation et/ou la position des corps magnétiques à l'intérieur du dépôt, le champ magnétique étant appliqué de manière à former au moins un motif sur la composition.

**20.** Kit selon la revendication 19, dans lequel la composition de maquillage comprend en outre au moins un polymère filmogène.

**21.** Kit selon la revendication 19 ou 20, dans lequel le dispositif magnétique comporte un aimant permanent.

**22.** Kit selon l'une des revendications 19 à 21, dans lequel la composition est à appliquer sur les lèvres.

**23.** Kit selon l'une quelconque des revendications 19 à 22, dans lequel la composition est contenue dans un dispositif de conditionnement et d'application comportant un récipient (2) et un applicateur (3).

**24.** Kit selon la revendication précédente, dans lequel l'applicateur comporte une tige (5) munie à une extrémité d'un organe d'application et à l'autre extrémité d'un organe de préhension.

**25.** Kit selon l'une quelconque des revendications 19 à 24, dans lequel le solvant volatil comprend au moins une huile volatile.

**Patentansprüche**

**1.** Verfahren zum Schminken von keratinischen Substanzen, insbesondere Haut, Lippen oder Phaneren, umfassend die folgenden Schritte:

(a) Aufbringen einer Schminkzusammensetzung auf die keratinischen Substanzen mittels eines nicht-magnetischen Applikators, wobei die Schminkzusammensetzung umfasst:

  (i) mindestens ein flüchtiges Lösungsmittel,
  (ii) Teilchen mit einer magnetischen Suszeptibilität von nicht Null,

(b) Aussetzen der Abscheidung einem Magnetfeld, derart dass die Orientierung modifiziert und/oder mindestens

bestimmte der Teilchen mit einer magnetischen Suszeptibilität von nicht Null bewegt werden,
wobei das Magnetfeld so angelegt wird, dass mindestens ein Muster auf der Zusammensetzung ausgebildet wird.

**2.** Verfahren nach Anspruch 1, wobei die Zusammensetzung mindestens ein filmbildendes Polymer umfasst.

**3.** Verfahren nach Anspruch 2, wobei das filmbildende Polymer ein Blockpolymer ist.

**4.** Verfahren nach Anspruch 3, wobei das Blockpolymer mindestens drei distinkte Sequenzen umfasst.

**5.** Verfahren nach einem der Ansprüche 1 bis 4, wobei das flüchtige Lösungsmittel ein organisches Lösungsmittel ist.

**6.** Verfahren nach Anspruch 5, wobei das flüchtige Lösungsmittel mindestens ein flüchtiges Öl umfasst.

**7.** Verfahren nach Anspruch 6, wobei das flüchtige Öl ein Kohlenwasserstofföl ist.

**8.** Verfahren nach Anspruch 6, wobei das flüchtige Öl ein Silikonöl ist.

**9.** Verfahren nach Anspruch 7, wobei das flüchtige Öl Isododecan umfasst.

**10.** Verfahren nach einem der Ansprüche 1 bis 9, wobei die Teilchen mit einer magnetischen Suszeptibilität von nicht Null Pigmente sind.

**11.** Verfahren nach einem der vorangehenden Ansprüche, wobei die magnetischen Teilchen metallisches Eisen, insbesondere Weicheisen umfassen.

**12.** Verfahren nach einem der vorangehenden Ansprüche, wobei das Magnetfeld durch einen Permantentmagneten ausgeübt wird.

**13.** Verfahren nach einem der Ansprüche 1 bis 11, wobei das Magnetfeld durch einen Elektromagneten ausgeübt wird.

**14.** Verfahren 12 oder 13, wobei das Magnetfeld drehbar ist.

**15.** Verfahren nach einem der vorangehenden Ansprüche, wobei mindestens ein Bereich des Trägers, der mit der Zusammensetzung überzogen ist, dem Magnetfeld nicht ausgesetzt ist.

**16.** Verfahren nach einem der vorangehenden Ansprüche, wobei die Abscheidung nach dem Anlegen eines Magnetfelds trocken gelassen wird.

**17.** Verfahren Anspruch 1, wobei die Abscheidung auf den Lippen gebildet wird.

**18.** Verfahren Anspruch 1, wobei die Abscheidung auf den Nägeln gebildet wird.

**19.** Kit zum Schminken der keratinischen Substanzen, zur Durchführung eines Verfahrens nach Anspruch 1, umfassend:

- eine Schminkzusammensetzung, umfassend:

  (i) mindestens ein flüchtiges Lösungsmittel
  (ii) Teilchen mit einer magnetischen Suszeptibilität von nicht Null,

- einen nicht magnetischen kosmetischen Applikator,
- eine magnetische Vorrichtung, die ermöglicht, dass ein Magnetfeld erzeugt wird,
- wobei die magnetische Vorrichtung dazu ausgelegt ist, ein empfindliches Magnetfeld zu erzeugen, wenn die mit der Abscheidung der Zusammensetzung bedeckten keratinischen Substanzen in das Magnetfeld eingebracht werden, dass die Orientierung und/oder die Position der Magnetkörper im Inneren der Abscheidung modifiziert werden, wobei das Magnetfeld so angelegt wird, dass mindestens ein Muster auf der Zusammensetzung ausgebildet wird,.

20. Kit nach Anspruch 19, wobei die Schminkzusammensetzung weiterhin mindestens ein filmbildendes Polymer umfasst.

21. Kit nach Anspruch 19 oder 20, wobei die magnetische Vorrichtung einen Permanentmagneten umfasst.

22. Kit nach einem der Ansprüche 19 bis 21, wobei die Zusammensetzung zum Auftragen auf die Lippen ist.

23. Kit nach einem der Ansprüche 19 bis 22, wobei die Zusammensetzung in einer Vorrichtung zum Verpacken und Aufbringen umfassend einen Behälter (2) und einen Applikator 3) enthalten ist.

24. Kit nach einem dem vorangehenden Ansprüche, wobei der Applikator einen Stab (5) umfasst, der an einem Ende mit einer Applikationseinrichtung und am anderen Ende mit einer Griffeinrichtung versehen ist.

25. Kit nach einem der Ansprüche 19 bis 24, wobei das flüchtige Lösungsmittel mindestens ein flüchtiges Öl umfasst.

## Claims

1. A method for making up keratinous materials, notably, skin, lips and appendages, comprising the following steps:

   (a) applying on the keratinous materials by means of a non-magnetic cosmetic applicator, a make-up composition comprising

      (i) at least one volatile solvent,
      (ii) particles having non-zero magnetic susceptibility,

   (b) subjecting the deposit to a magnetic field so as to modify the orientation and/or to displace at least some of said particles with non-zero magnetic susceptibility,

   the magnetic field being applied so as to form at least one pattern on the composition.

2. The method according to claim 1, wherein the composition comprises at least one film-forming polymer.

3. The method according to claim 2, wherein the film-forming polymer is a sequenced polymer.

4. The method according to claim 3, wherein the sequenced polymer includes at least three distinct sequences.

5. The method according to any of claims 1 to 4, wherein
the volatile solvent is an organic solvent.

6. The method according to claim 5, wherein the volatile solvent comprises at least one volatile oil.

7. The method according to claim 6, wherein the volatile oil is a hydrocarbon oil.

8. The method according to claim 6, wherein the volatile oil is a silicone oil.

9. The method according to claim 7, wherein the volatile oil comprises isododecane.

10. The method according to any of claims 1 to 9, wherein the particles having non-zero magnetic susceptibility are pigments.

11. The method according to any of the preceding claims, wherein the magnetic particles include metal iron, notably soft iron.

12. The method according to any of the preceding claims, wherein the magnetic field is exerted by a permanent magnet.

13. The method according to any of claims 1 to 11, wherein the magnetic field is exerted by an electromagnet.

**14.** The method according to claim 12 or 13, wherein the magnetic field is rotary.

**15.** The method according to any of the preceding claims, wherein at least one region of the support coated with the composition is not exposed to the magnetic field.

**16.** The method according to any of the preceding claims, wherein the deposit is left to dry after applying the magnetic field.

**17.** The method according to claim 1, wherein the deposit is formed on the lips.

**18.** The method according to claim 1, wherein the deposit is formed on the nails.

**19.** A make-up kit for keratinous materials in order to apply a method according to claim 1, including:

a make-up composition comprising:

(i) at least one volatile solvent,
(ii) particles having non-zero magnetic susceptibility,

a non-magnetic cosmetic applicator,
a magnetic device allowing generation of a magnetic field, the magnetic device being capable of creating a magnetic field which may, when the keratinous materials covered with a deposit of said composition are introduced into said magnetic field, modify the orientation and/or the position of the magnetic bodies inside the deposit, the magnetic field being applied so as to form at least one pattern on the composition.

**20.** The kit according to claim 19, wherein the make-up composition further comprises at least one film-forming polymer.

**21.** The kit according to claim 19 or 20, wherein the magnetic device includes a permanent magnet.

**22.** The kit according to one of claims 19 to 21, wherein the composition is to be applied on the lips.

**23.** The kit according to any of claims 19 to 22, wherein the composition is contained in a conditioning and application device including a container (2) and an applicator (3).

**24.** The kit according to the preceding claim, wherein the applicator includes a rod (5) provided at one end with an application member and at the other end with a gripping member.

**25.** The kit according to any of claims 19 to 24, wherein the volatile solvent comprises at least one volatile oil.

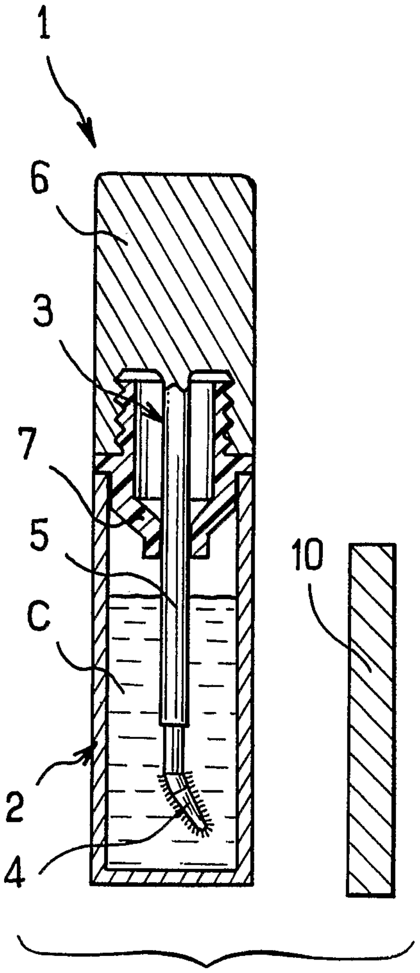


FIG_1

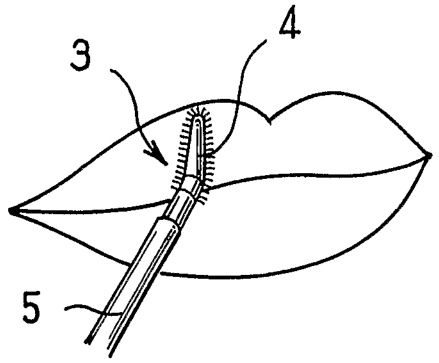


FIG_2

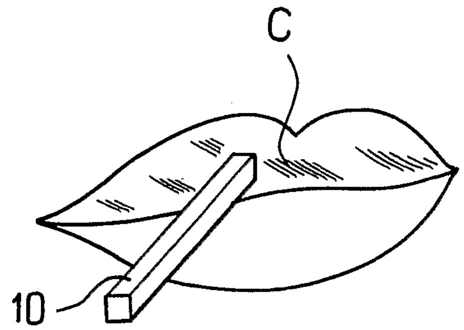


FIG_3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1264562 A
- EP 749747 A
- EP 895467 A
- EP 96459 A
- US 5625005 A
- US 5725882 A
- US 5209924 A
- US 4972037 A
- US 4981903 A
- US 4981902 A
- US 5468477 A
- US 5219560 A
- EP 0388582 A
- US 5948393 A
- EP 0815836 A
- US 5849318 A
- WO OS129880 A
- WO OS129881 A
- WO OS84383 A
- FR 2232303 A
- FR 0113920
- US 6074654 A
- US 5874069 A
- US 5919441 A
- US 6051216 A
- US 5981680 A
- US 5162410 A
- US 330747 A
- US 5451610 A
- US 5188899 A
- EP 080976 A
- FR 2077143
- FR 2393573
- US 3589578 A
- US 4031307 A
- US 4131576 A
- US 4693935 A
- US 4728571 A
- EP 0412704 A
- EP 0412707 A
- EP 0640105 A
- WO 9500578 A
- EP 0582152 A
- WO 9323009 A
- WO 9503776 A
- US 5061481 A
- US 5849275 A
- US 6033650 A
- WO 9323446 A
- WO 9506078 A
- US 20030031870 A
- US 6428773 B
- EP 1184426 A2
- EP 1184426 A
- EP 1217046 A
- JP 3286463 B


### Littérature non-brevet citée dans la description


- Solubility parameter values. **HANSEN ; ERIC A.GRRULKE.** Polymer Handbook. 519-559
- **C.M.HANSEN.** The three dimensional solubility parameters. *J.Paint Technol.,* 1967, vol. 39, 105
- **GILLMAN K.F.** *Polymer Letters,* 1967, vol. 5, 477-481
- Polymer Handbook. John Wiley, 1989
- **E.M. FURST ; C. SUZUKI ; M. FERMIGIER ; A.P. GAST.** Permanently linked monodisperse paramagnetic chains. *Langmuir,* 1998, vol. 14, 7334-7336
- **M. FERMIGIER ; Y. GRASSELLI.** Suspensions de particules magnétiques. *Bulletin de la SFP (105),* Juillet 1996
- **C. GOUBAULT ; P. JOP ; M. FERMIGIER ; J. BAUDRY ; E. BERTRAND ; J. BIBETTE.** Flexible magnetic filaments as micromechanical sensors. *Phys. Rev. Lett.,* 2003, vol. 91 (26), 260802-1, 260802-4
- **ALBERTO ARGOITIA ; MATT WITZMAN.** Pigments Exhibiting Diffractive Effects. *Society of Vacuum coaters, 45th Annual Technical Conference Proceedings 2002,* 2002
- International Cosmetic Ingredient Dictionnary and Handbook. The Cosmetic, Toiletry, and Fragrance Association, 1997, 371-386, 524-528